# EUROPEAN PATENT SPECIFICATION

(11) **EP 3 448 401 B1**
(45) Date of publication and mention of the grant of the patent: **27.10.2021**
(21) Application number: 17790621.1
(22) Date of filing: 29.04.2017
(51) Int. Cl.: A61K 35/76, C12N 7/00, A61K 48/00, A61K 39/00

(54) **HSV VECTORS WITH ENHANCED REPLICATION IN CANCER CELLS**
HSV-VEKTOREN MIT VERBESSERTE REPLIKATION IN KREBSZELLEN
VECTEURS DU VHS À RÉPLICATION FAVORISÉE DANS DES CELLULES CANCÉREUSES

(30) Priority: 29.04.2016 US 201662329877 P
(43) Date of publication of application: 06.03.2019
(73) Proprietor: Virogin Biotech Canada Ltd, Vancouver, British Columbia V6S 2L9 (CA)
(72) Inventor: JIA, William, Burnaby British Columbia V5B 3J9 (CA); CHOULJENKO, Dmitry, Vancouver British Columbia V6R 2J4 (CA); BU, Xuexian, Surrey British Columbia V3T 1N9 (CA); LIU, Guoyu, Richmond British Columbia V6Y 3J5 (CA)
(74) Representative: Forresters IP LLP
(86) International application number: PCT/US2017/030308
(87) International publication number: WO 2017/190112

(56) References cited:
- WO-A1-2015/066042
- US-A1- 2014 093 884
- ZAHID M. DELWAR ET AL: "Tumour-specific triple-regulated oncolytic herpes virus to target glioma", ONCOTARGET, vol. 7, no. 19, 10 May 2016 (2016-05-10), XP055627619, DOI: 10.18632/oncotarget.8637
- CODY ET AL.: 'Histone Deacetylase Inhibitors Improve the Replication of Oncolytic Herpes Simplex Virus in Breast Cancer Cells' PLOS ONE vol. 9, no. 3, 2014, page E92919, XP055436775
- POST ET AL.: 'Replicative oncolytic herpes simplex viruses in combination cancer therapies' CURR GENE THER vol. 4, no. 1, 2004, pages 41 - 51, XP055436776

### SEQUENCE LISTING

<110> Virogin Biotech Canada Ltd
<120> A HSV-1 VIRUS WITH ENHANCED REPLICATION IN CANCER CELLS
<130> VIRO.402PC
<150> US 62/329,877
   <151> 2016-04-29
<160> 40
<170> PatentIn version 3.5
<210> 1
   <211> 10
   <212> DNA
   <213> Homo sapiens
<400> 1
   gggaatttcc 10
<210> 2
   <211> 39
   <212> DNA
   <213> Homo sapiens
<400> 2
   ctacagaggt gcatattaac agagcttttg tcctggaga 39
<210> 3
   <211> 2894
   <212> DNA
   <213> Herpes Simplex Virus
<400> 3
   atgctcgccg tccgttccct gcagcacctc tcaaccgtcg tcttgataac ggcgtacggc        60
   ctcgtgctcg tgtggtacac cgtcttcggt gccagtccgc tgcaccgatg tatttacgcg       120
   gtacgcccca ccggcaccaa caacgacacc gccctcgtgt ggatgaaaat gaaccagacc       180
   ctattgtttc tgggggcccc gacgcacccc cccaacgggg gctggcgcaa ccacgcccat       240
   atctgctacg ccaatcttat cgcgggtagg gtcgtgccct tccaggtccc acctgacgcc       300
   atgaatcgtc ggatcatgaa cgtccacgag gcagttaact gtctggagac cctatggtac       360
   acacgggtgc gtctggtggt cgtagggtgg ttcctgtatc tggcgttcgt cgccctccac       420
   caacgccgat gtatgtttgg cgtcgtgagt cccgcccaca agatggtggc cccggccacc       480
   tacctcttga actacgcagg ccgcatcgta tcgagcgtgt tcctgcagta cccctacacg       540
   aaaattaccc gcctgctctg cgagctgtcg gtccagcggc aaaacctggt tcagttgttt       600
   gagacggacc cggtcacctt cttgtaccac cgccccgcca tcggggtcat cgtaggctgc       660
   gagttgatgc tacgctttgt ggccgtgggt ctcatcgtcg gcaccgcttt catatcccgg       720
   ggggcatgtg caatcacata ccccctgttt ctgaccatca ccacctggtg ttttgtctcc 780
   accatcggcc tgacagagct gtattgtatt ctgcggcggg gcccggcccc caagaacgca 840
   gacaaggccg ccgccccggg gcgatccaag gggctgtcgg gcgtctgcgg gcgctgctgt 900
   tccatcatcc tctcgggcat cgcagtgcga ttgtgttata tcgccgtggt ggccggggtg 960
   gtgctcgtgg cgcttcacta cgagcaggag atccagaggc gcctgtttga tgtatgacgt 1020
   cactacagag gtgcatatta acagagcttt tgtcctggag aaagcttcgc gttctttgaa 1080
   agcagtcgag ggggcgctag atgtgggcag ggacgagctg gcgcggcgtc gctgggtgca 1140
   ccgcgaccac gggcagagcc acgcggcggg aggactacaa ctcccggcac accccgcgcc 1200
   gccccgcctc tactcccaga aggccgcggg gggtggaccg cctaagaggg cgtgcgctcc 1260
   cgacatgccc cgcggcgcgc cattaaccgc cagatttgaa tcgcgggacc cgttggcaga 1320
   ggtggcggcg gcggcaaagg gcgaattgat ccgtcatggc gactgacatt gatatgctaa 1380
   ttgacctcgg cctggacctc tccgacagcg atctggacga ggaccccccc gagccggcgg 1440
   agagccgccg cgacgacctg gaatcggaca gcagcgggga gtgttcctcg tcggacgagg 1500
   acatggaaga cccccacgga gaggacggac cggagccgat actcgacgcc gctcgcccgg 1560
   cggtccgccc gtctcgtcca gaagaccccg gcgtacccag cacccagacg cctcgtccga 1620
   cggagcggca gggccccaac gatcctcaac cagcgcccca cagtgtgtgg tcgcgcctcg 1680
   gggcccggcg accgtcttgc tcccccgagc agcacggggg caaggtggcc cgcctccaac 1740
   ccccaccgac caaagcccag cctgcccgcg gcggacgccg tgggcgtcgc aggggtcggg 1800
   gtcgcggtgg tcccggggct gccgatggtt tgtcggaccc ccgccggcgt gcccccagaa 1860
   ccaatcgcaa ccctggggga ccccgccccg gggcggggtg gacggacggc cccggcgccc 1920
   cccatggcga ggcgtggcgc ggcagtgagc agcccgaccc acccggaggc cagcggacac 1980
   ggggcgtgcg ccaagcaccc cccccgctaa tgacgctggc gattgccccc ccgcccgcgg 2040
   acccccgcgc cccggccccg gagcgaaagg cgcccgccgc cgacaccatc gacgccacca 2100
   cgcggttggt cctgcgctcc atctccgagc gcgcggcggt cgaccgcatc agcgagagct 2160
   ttggccgcag cgcacaggtc atgcacgacc cctttggggg gcagccgttt cccgccgcga 2220
   atagcccctg ggccccggtg ctggcgggcc aaggagggcc ctttgacgcc gagaccagac 2280
   gggtctcctg ggaaaccttg gtcgcccacg gcccgagcct ctatcgcact tttgccggca 2340
   atcctcgggc cgcatcgacc gccaaggcca tgcgcgactg cgtgctgcgc caagaaaatt 2400
   tcatcgaggc gctggcctcc gccgacgaga cgctggcgtg gtgcaagatg tgcatccacc 2460
   acaacctgcc gctgcgcccc caggacccca ttatcgggac ggccgcggct gtgctggata 2520
   acctcgccac gcgcctgcgg ccctttctcc agtgctacct gaaggcgcga ggcctgtgcg 2580
   gcctggacga actgtgttcg cggcggcgtc tggcggacat taaggacatt gcatccttcg 2640
   tgtttgtcat tctggccagg ctcgccaacc gcgtcgagcg tggcgtcgcg gagatcgact      2700
   acgcgaccct tggtgtcggg gtcggagaga agatgcattt ctacctcccc ggggcctgca      2760
   tggcgggcct gatcgaaatc ctagacacgc accgccagga gtgttcgagt cgtgtctgcg      2820
   agttgacggc cagtcacatc gtcgcccccc cgtacgtgca cggcaaatat ttttattgca      2880
   actccctgtt ttag                                                        2894
<210> 4
   <211> 3185
   <212> DNA
   <213> Herpes Simplex Virus
<400> 4
   atgctcgccg tccgttccct gcagcacctc tcaaccgtcg tcttgataac ggcgtacggc 60
   ctcgtgctcg tgtggtacac cgtcttcggt gccagtccgc tgcaccgatg tatttacgcg 120
   gtacgcccca ccggcaccaa caacgacacc gccctcgtgt ggatgaaaat gaaccagacc 180
   ctattgtttc tgggggcccc gacgcacccc cccaacgggg gctggcgcaa ccacgcccat 240
   atctgctacg ccaatcttat cgcgggtagg gtcgtgccct tccaggtccc acctgacgcc 300
   atgaatcgtc ggatcatgaa cgtccacgag gcagttaact gtctggagac cctatggtac 360
   acacgggtgc gtctggtggt cgtagggtgg ttcctgtatc tggcgttcgt cgccctccac 420
   caacgccgat gtatgtttgg cgtcgtgagt cccgcccaca agatggtggc cccggccacc 480
   tacctcttga actacgcagg ccgcatcgta tcgagcgtgt tcctgcagta cccctacacg 540
   aaaattaccc gcctgctctg cgagctgtcg gtccagcggc aaaacctggt tcagttgttt 600
   gagacggacc cggtcacctt cttgtaccac cgccccgcca tcggggtcat cgtaggctgc 660
   gagttgatgc tacgctttgt ggccgtgggt ctcatcgtcg gcaccgcttt catatcccgg 720
   ggggcatgtg cgatcacata ccccctgttt ctgaccatca ccacctggtg ttttgtctcc 780
   accatcggcc tgacagagct gtattgtatt ctgcggcggg gcccggcccc caagaacgca 840
   gacaaggccg ccgccccggg gcgatccaag ggctgtcggg gcgtctgcgg gcgctgctgt 900
   tccatcatcc tctcgggcat cgcagtgcga ttgtgttata tcgccgtggt ggccggggtg 960
   gtgctcgtgg cgcttcacta cgagcaggag atccagaggc gcctgtttga tgtatgacgt 1020
   cacatccagg ccggcggaaa ccgtaacggc atatgcaaat tggaaactgt cctgtcttgg 1080
   ggcccaccca cccgacgcgt catatgcaaa tgaaaatcgg tcccccgagg ccacgtgtag 1140
   cctggatccc aacgaccccg cccatgggtc ccaattggcc gtcccgttac caagaccaac 1200
   ccagccagca tatccacccc cgcccgggtc cccgcggaag cggaacgggg tatgtgatat 1260
   gctaattaaa tacgggaatt tccggggact ttccgggaat ttccggggac tttccgggaa 1320
   tttccctaca gaggtgcata ttaacagagc ttttgtcctg gagaatgcca cgtacttatg 1380
   gtgtctgatt ggtccttgtc tgtgccggag gtggggcggg ggccccgccc ggggggcgga 1440
   acgaggaggg gtttgggaga gccggccccg gcaccacggg tataaggaca tccaccaccc 1500
   ggccggtggt ggtgtgcagc cgtgttccaa ccacggtcac gcttcggtgc ctctccccga 1560
   ttcgggcccg gtcgctcgct accggtgcgc caccaccaga ggccatatcc gacaccccag 1620
   ccccgacggc agccgacagc ccggtcatgg cgactgacat tgatatgcta attgacctcg 1680
   gcctggacct ctccgacagc gatctggacg aggacccccc cgagccggcg gagagccgcc 1740
   gcgacgacct ggaatcggac agcagcgggg agtgttcctc gtcggacgag gacatggaag 1800
   acccccacgg agaggacgga ccggagccga tactcgacgc cgctcgcccg gcggtccgcc 1860
   cgtctcgtcc agaagacccc ggcgtaccca gcacccagac gcctcgtccg acggagcggc 1920
   agggccccaa cgatcctcaa ccagcgcccc acagtgtgtg gtcgcgcctc ggggcccggc 1980
   gaccgtcttg ctcccccgag cagcacgggg gcaaggtggc ccgcctccaa cccccaccga 2040
   ccaaagccca gcctgcccgc ggcggacgcc gtgggcgtcg caggggtcgg ggtcgcggtg 2100
   gtcccggggc tgccgatggt ttgtcggacc cccgccggcg tgcccccaga accaatcgca 2160
   accctggggg accccgcccc ggggcggggt ggacggacgg ccccggcgcc ccccatggcg 2220
   aggcgtggcg cggcagtgag cagcccgacc cacccggagg ccagcggaca cggggcgtgc 2280
   gccaagcacc ccccccgcta atgacgctgg cgattgcccc cccgcccgcg gacccccgcg 2340
   ccccggcccc ggagcgaaag gcgcccgccg ccgacaccat cgacgccacc acgcggttgg 2400
   tcctgcgctc catctccgag cgcgcggcgg tcgaccgcat cagcgagagc tttggccgca 2460
   gcgcacaggt catgcacgac ccctttgggg ggcagccgtt tcccgccgcg aatagcccct 2520
   gggccccggt gctggcgggc caaggagggc cctttgacgc cgagaccaga cgggtctcct 2580
   gggaaacctt ggtcgcccac ggcccgagcc tctatcgcac ttttgccggc aatcctcggg 2640
   ccgcatcgac cgccaaggcc atgcgcgact gcgtgctgcg ccaagaaaat ttcatcgagg 2700
   cgctggcctc cgccgacgag acgctggcgt ggtgcaagat gtgcatccac cacaacctgc 2760
   cgctgcgccc ccaggacccc attatcggga cggccgcggc tgtgctggat aacctcgcca 2820
   cgcgcctgcg gccctttctc cagtgctacc tgaaggcgcg aggcctgtgc ggcctggacg 2880
   aactgtgttc gcggcggcgt ctggcggaca ttaaggacat tgcatccttc gtgtttgtca 2940
   ttctggccag gctcgccaac cgcgtcgagc gtggcgtcgc ggagatcgac tacgcgaccc 3000
   ttggtgtcgg ggtcggagag aagatgcatt tctacctccc cggggcctgc atggcgggcc 3060
   tgatcgaaat cctagacacg caccgccagg agtgttcgag tcgtgtctgc gagttgacgg 3120
   ccagtcacat cgtcgccccc ccgtacgtgc acggcaaata tttttattgc aactccctgt 3180
   tttag 3185
<210> 5
   <211> 3185
   <212> DNA
   <213> Herpes Simplex Virus
<400> 5
   atgctcgccg tccgttccct gcagcacctc tcaaccgtcg tcttgataac ggcgtacggc 60
   ctcgtgctcg tgtggtacac cgtcttcggt gccagtccgc tgcaccgatg tatttacgcg 120
   gtacgcccca ccggcaccaa caacgacacc gccctcgtgt ggatgaaaat gaaccagacc 180
   ctattgtttc tgggggcccc gacgcacccc cccaacgggg gctggcgcaa ccacgcccat 240
   atctgctacg ccaatcttat cgcgggtagg gtcgtgccct tccaggtccc acctgacgcc 300
   atgaatcgtc ggatcatgaa cgtccacgag gcagttaact gtctggagac cctatggtac 360
   acacgggtgc gtctggtggt cgtagggtgg ttcctgtatc tggcgttcgt cgccctccac 420
   caacgccgat gtatgtttgg cgtcgtgagt cccgcccaca agatggtggc cccggccacc 480
   tacctcttga actacgcagg ccgcatcgta tcgagcgtgt tcctgcagta cccctacacg 540
   aaaattaccc gcctgctctg cgagctgtcg gtccagcggc aaaacctggt tcagttgttt 600
   gagacggacc cggtcacctt cttgtaccac cgccccgcca tcggggtcat cgtaggctgc 660
   gagttgatgc tacgctttgt ggccgtgggt ctcatcgtcg gcaccgcttt catatcccgg 720
   ggggcatgtg cgatcacata ccccctgttt ctgaccatca ccacctggtg ttttgtctcc 780
   accatcggcc tgacagagct gtattgtatt ctgcggcggg gcccggcccc caagaacgca 840
   gacaaggccg ccgccccggg gcgatccaag gggctgtcgg gcgtctgcgg gcgctgctgt 900
   tccatcatcc tctcgggcat cgcagtgcga ttgtgttata tcgccgtggt ggccggggtg 960
   gtgctcgtgg cgcttcacta cgagcaggag atccagaggc gcctgtttga tgtatgacgt 1020
   cacatccagg ccggcggaaa ccgtaacggc atatgcaaat tggaaactgt cctgtcttgg 1080
   ggcccaccca cccgacgcgt catatgcaaa tgaaaatcgg tcccccgagg ccacgtgtag 1140
   cctggatccc aacgaccccg cccatgggtc ccaattggcc gtcccgttac caagaccaac 1200
   ccagccagca tatccacccc cgcccgggtc cccgcggaag cggaacggtg tatgtgatat 1260
   gctaattaaa tacatgccac gtacttatgg tgtctgattg gtccttgtct gtgccggagg 1320
   ggaatttccg gggactttcc gggaatttcc ggggactttc cgggaatttc cctacagagg 1380
   tgcatattaa cagagctttt gtcctggaga gtggggcggg ggccccgccc ggggggcgga 1440
   acgaggaggg gtttgggaga gccggccccg gcaccacggg tataaggaca tccaccaccc 1500
   ggccggtggt ggtgtgcagc cgtgttccaa ccacggtcac gcttcggtgc ctctccccga 1560
   ttcgggcccg gtcgctcgct accggtgcgc caccaccaga ggccatatcc gacaccccag 1620
   ccccgacggc agccgacagc ccggtcatgg cgactgacat tgatatgcta attgacctcg 1680
   gcctggacct ctccgacagc gatctggacg aggacccccc cgagccggcg gagagccgcc 1740
   gcgacgacct ggaatcggac agcagcgggg agtgttcctc gtcggacgag gacatggaag 1800
   acccccacgg agaggacgga ccggagccga tactcgacgc cgctcgcccg gcggtccgcc 1860
   cgtctcgtcc agaagacccc ggcgtaccca gcacccagac gcctcgtccg acggagcggc 1920
   agggccccaa cgatcctcaa ccagcgcccc acagtgtgtg gtcgcgcctc ggggcccggc 1980
   gaccgtcttg ctcccccgag cagcacgggg gcaaggtggc ccgcctccaa cccccaccga 2040
   ccaaagccca gcctgcccgc ggcggacgcc gtgggcgtcg caggggtcgg ggtcgcggtg 2100
   gtcccggggc tgccgatggt ttgtcggacc cccgccggcg tgcccccaga accaatcgca 2160
   accctggggg accccgcccc ggggcggggt ggacggacgg ccccggcgcc ccccatggcg 2220
   aggcgtggcg cggcagtgag cagcccgacc cacccggagg ccagcggaca cggggcgtgc 2280
   gccaagcacc ccccccgcta atgacgctgg cgattgcccc cccgcccgcg gacccccgcg 2340
   ccccggcccc ggagcgaaag gcgcccgccg ccgacaccat cgacgccacc acgcggttgg 2400
   tcctgcgctc catctccgag cgcgcggcgg tcgaccgcat cagcgagagc tttggccgca 2460
   gcgcacaggt catgcacgac ccctttgggg ggcagccgtt tcccgccgcg aatagcccct 2520
   gggccccggt gctggcgggc caaggagggc cctttgacgc cgagaccaga cgggtctcct 2580
   gggaaacctt ggtcgcccac ggcccgagcc tctatcgcac ttttgccggc aatcctcggg 2640
   ccgcatcgac cgccaaggcc atgcgcgact gcgtgctgcg ccaagaaaat ttcatcgagg 2700
   cgctggcctc cgccgacgag acgctggcgt ggtgcaagat gtgcatccac cacaacctgc 2760
   cgctgcgccc ccaggacccc attatcggga cggccgcggc tgtgctggat aacctcgcca 2820
   cgcgcctgcg gccctttctc cagtgctacc tgaaggcgcg aggcctgtgc ggcctggacg 2880
   aactgtgttc gcggcggcgt ctggcggaca ttaaggacat tgcatccttc gtgtttgtca 2940
   ttctggccag gctcgccaac cgcgtcgagc gtggcgtcgc ggagatcgac tacgcgaccc 3000
   ttggtgtcgg ggtcggagag aagatgcatt tctacctccc cggggcctgc atggcgggcc 3060
   tgatcgaaat cctagacacg caccgccagg agtgttcgag tcgtgtctgc gagttgacgg 3120
   ccagtcacat cgtcgccccc ccgtacgtgc acggcaaata tttttattgc aactccctgt 3180
   tttag 3185
<210> 6
   <211> 1230
   <212> DNA
   <213> Herpes Simplex Virus
<400> 6
   gtgacgattc ccccaatggc cgcgcgtccc aggggaggca ggcccaccgc ggggcggccc      60
   cgtccccggg gaccaacccg gcgcccccaa agaatatcat tagcatgcac ggcccggccc     120
   ccgatttggg ggcccaaccc ggtgtccccc aaagaacccc attagcatgc ccctcccgcc     180
   gacgcaacag gggcttggcc tgcgtcggtg ccccggggct tcccgccttc ccgaagaaac     240
   tcattaccat acccggaacc ccaggggacc aatgcgggtt cattgagcga cccgcgggcc     300
   aatgcgcgag ggccgtgtgg ttccgccaaa aaagcaatta gcataacccg gaaccccagg     360
   ggagtggtta cgcgcggcgc gggaggcggg gaataccggg gttgcccatt aagggccgcg     420
   ggaattgccg gaagcgggaa gggcggccgg ggccgcccat tagttacctg ggactgtgcg     480
   gttgggacgg cgcccgtggg cccgggcggc cgggggcggc gggggccgcg atggcggcgg     540
   cggcgggcca tggagacaga gagcgtgccg gggtggtaga gtttgacagg caagcatgtg     600
   cgtgcagagg cgagtagtgc ttgcctgtct aactcgctag tctcggccgc ggggggcccg     660
   ggctgcccgc cgccgccgct ttaaagggcc gcgcgcgacc cccggggggt gtgttttggg     720
   gggggcccgt tttcggggtc tggccgctcc tccccccgct cctccccccg ctcctccccc     780
   cgctcctccc cccgctcctc cccccgctcc tccccccgct cctccccccg ctcctccccc     840
   cgctcctccc cccgctcctc cccccgctcc tccccccgct cctccccccg ctcctccccc     900
   cgctcctccc cccgctcctc cccccgctcc tccccccgct cctccccccg ctcctccccc     960
   cgctcccgcg gccccgcccc ccacgcccgc cgcgcgcgcg cacgccgccc ggaccgccgc    1020
   ccgccttttt tgcgcgcgcg cgcgcccgcg gggggcccgg gctgccacag gtgaaaccaa    1080
   cagagcacgg cgcactccgc acgtcacacg tcacgtcatc caccacacct gcccaacaac    1140
   acaactcaca gcgacaactc accgcgcaac aactcctgtt cctcatccac acgtcaccgc    1200
   gcacctcccg ctcctccaga cgtaccccgg                                    1230
<210> 7
   <211> 3185
   <212> DNA
   <213> Herpes Simplex Virus
<400> 7
   atgctcgccg tccgttccct gcagcacctc tcaaccgtcg tcttgataac ggcgtacggc      60
   ctcgtgctcg tgtggtacac cgtcttcggt gccagtccgc tgcaccgatg tatttacgcg     120
   gtacgcccca ccggcaccaa caacgacacc gccctcgtgt ggatgaaaat gaaccagacc     180
   ctattgtttc tgggggcccc gacgcacccc cccaacgggg gctggcgcaa ccacgcccat     240
   atctgctacg ccaatcttat cgcgggtagg gtcgtgccct tccaggtccc acctgacgcc     300
   atgaatcgtc ggatcatgaa cgtccacgag gcagttaact gtctggagac cctatggtac     360
   acacgggtgc gtctggtggt cgtagggtgg ttcctgtatc tggcgttcgt cgccctccac     420
   caacgccgat gtatgtttgg cgtcgtgagt cccgcccaca agatggtggc cccggccacc     480
   tacctcttga actacgcagg ccgcatcgta tcgagcgtgt tcctgcagta cccctacacg     540
   aaaattaccc gcctgctctg cgagctgtcg gtccagcggc aaaacctggt tcagttgttt 600
   gagacggacc cggtcacctt cttgtaccac cgccccgcca tcggggtcat cgtaggctgc 660
   gagttgatgc tacgctttgt ggccgtgggt ctcatcgtcg gcaccgcttt catatcccgg 720
   ggggcatgtg cgatcacata ccccctgttt ctgaccatca ccacctggtg ttttgtctcc 780
   accatcggcc tgacagagct gtattgtatt ctgcggcggg gcccggcccc caagaacgca 840
   gacaaggccg ccgccccggg gcgatccaag gggctgtcgg gcgtctgcgg gcgctgctgt 900
   tccatcatcc tctcgggcat cgcagtgcga ttgtgttata tcgccgtggt ggccggggtg 960
   gtgctcgtgg cgcttcacta cgagcaggag atccagaggc gcctgtttga tgtatgacgt 1020
   cacatccagg ccggcggaaa ccgtaacggc atatgcaaat tggaaactgt cctgtcttgg 1080
   ggcccaccca cccgacgcgt catatgcaaa tgaaaatcgg tcccccgagg ccacgtgtag 1140
   cctggatccc aacgaccccg cccatgggtc ccaattggcc gtcccgttac caagaccaac 1200
   ccagccagca tatccacccc cgcccgggtc cccgcggaag cggaacgggg tatgtgatat 1260
   gctaattaaa tacgggaatt tccggggact ttccgggaat ttccggggac tttccgggaa 1320
   tttccctaca gaggtgcata ttaacagagc ttttgtcctg gagaatgcca cgtacttatg 1380
   gtgtctgatt ggtccttgtc tgtgccggag gtggggcggg ggccccgccc ggggggcgga 1440
   acgaggaggg gtttgggaga gccggccccg gcaccacggg tataaggaca tccaccaccc 1500
   ggccggtggt ggtgtgcagc cgtgttccaa ccacggtcac gcttcggtgc ctctccccga 1560
   ttcgggcccg gtcgctcgct accggtgcgc caccaccaga ggccatatcc gacaccccag 1620
   ccccgacggc agccgacagc ccggtcatgg cgactgacat tgatatgcta attgacctcg 1680
   gcctggacct ctccgacagc gatctggacg aggacccccc cgagccggcg gagagccgcc 1740
   gcgacgacct ggaatcggac agcagcgggg agtgttcctc gtcggacgag gacatggaag 1800
   acccccacgg agaggacgga ccggagccga tactcgacgc cgctcgcccg gcggtccgcc 1860
   cgtctcgtcc agaagacccc ggcgtaccca gcacccagac gcctcgtccg acggagcggc 1920
   agggccccaa cgatcctcaa ccagcgcccc acagtgtgtg gtcgcgcctc ggggcccggc 1980
   gaccgtcttg ctcccccgag cagcacgggg gcaaggtggc ccgcctccaa cccccaccga 2040
   ccaaagccca gcctgcccgc ggcggacgcc gtgggcgtcg caggggtcgg ggtcgcggtg 2100
   gtcccggggc tgccgatggt ttgtcggacc cccgccggcg tgcccccaga accaatcgca 2160
   accctggggg accccgcccc ggggcggggt ggacggacgg ccccggcgcc ccccatggcg 2220
   aggcgtggcg cggcagtgag cagcccgacc cacccggagg ccagcggaca cggggcgtgc 2280
   gccaagcacc ccccccgcta atgacgctgg cgattgcccc cccgcccgcg gacccccgcg 2340
   ccccggcccc ggagcgaaag gcgcccgccg ccgacaccat cgacgccacc acgcggttgg 2400
   tcctgcgctc catctccgag cgcgcggcgg tcgaccgcat cagcgagagc tttggccgca 2460
   gcgcacaggt catgcacgac ccctttgggg ggcagccgtt tcccgccgcg aatagcccct 2520
   gggccccggt gctggcgggc caaggagggc cctttgacgc cgagaccaga cgggtctcct 2580
   gggaaacctt ggtcgcccac ggcccgagcc tctatcgcac ttttgccggc aatcctcggg 2640
   ccgcatcgac cgccaaggcc atgcgcgact gcgtgctgcg ccaagaaaat ttcatcgagg 2700
   cgctggcctc cgccgacgag acgctggcgt ggtgcaagat gtgcatccac cacaacctgc 2760
   cgctgcgccc ccaggacccc attatcggga cggccgcggc tgtgctggat aacctcgcca 2820
   cgcgcctgcg gccctttctc cagtgctacc tgaaggcgcg aggcctgtgc ggcctggacg 2880
   aactgtgttc gcggcggcgt ctggcggaca ttaaggacat tgcatccttc gtgtttgtca 2940
   ttctggccag gctcgccaac cgcgtcgagc gtggcgtcgc ggagatcgac tacgcgaccc 3000
   ttggtgtcgg ggtcggagag aagatgcatt tctacctccc cggggcctgc atggcgggcc 3060
   tgatcgaaat cctagacacg caccgccagg agtgttcgag tcgtgtctgc gagttgacgg 3120
   ccagtcacat cgtcgccccc ccgtacgtgc acggcaaata tttttattgc aactccctgt 3180
   tttag 3185
<210> 8
   <211> 3670
   <212> DNA
   <213> Herpes Simplex Virus
<400> 8
   atgtcgggcg tcgggggaga gggagttccc tctgcgcttg cgattctagc ctcgtggggc 60
   tggacgttcg acacgccaaa ccacgagtcg gggatatcgc cagatacgac tcccgcagat 120
   tccattcggg gtgccgctgt ggcctcacct gaccaacctt tacacggggg cccggaacgg 180
   gaggccacag cgccgtcttt ctccccaacg cgcgcggatg acggcccgcc ctgtaccgac 240
   gggccctacg tgacgtttga taccctgttt atggtgtcgt cgatcgacga attagggcgt 300
   cgccagctca cggacaccat ccgcaaggac ctgcggttgt cgctggccaa gtttagcatt 360
   gcgtgcacca agacctcctc gttttcggga aacgccccgc gccaccacag acgcggggcg 420
   ttccagcgcg gcacgcgggc gccgcgcagc aacaaaagcc tccagatgtt tgtgttgtgc 480
   aaacgcgccc acgccgctcg agtgcgagag cagcttcggg tcgttattca gtcccgcaag 540
   ccgcgcaagt attacacgcg atcttcggac gggcggctct gccccgccgt ccccgtgttc 600
   gtccacgagt tcgtctcgtc cgagccaatg cgcctccacc gagataacgt catgctggcc 660
   tcgggggccg agtaaccgcc cccccgcgcc accctcactg cccgtcgcgc gtgtttgatg 720
   ttaataaata acgcataaat ttggctggtt gtttgttgtc tttaatggac cgcccgcagg 780
   gggggtggca tttcagtgtc gggtgacgag cgcgatccgg ccgggagagg ctccggtgcc 840
   cgtcagtggg cagagcgcac atcgcccaca gtccccgaga agttgggggg aggggtcggc 900
   aattgaaccg gtgcctagag aaggtggcgc ggggtaaact gggaaagtga tgtcgtgtac 960
   tggctccgcc tttttcccga gggtggggga gaaccgtata taagtgcagt agtcgccgtg 1020
   aacgttcttt ttcgcaacgg gtttgccgcc agaacacagg taagtgccgt gtgtggttcc 1080
   cgcgggcctg gcctctttac gggttatggc ccttgcgtgc cttgaattac ttccacgccc 1140
   ctggctgcag tacgtgattc ttgatcccga gcttcgggtt ggaagtgggt gggagagttc 1200
   gaggccttgc gcttaaggag ccccttcgcc tcgtgcttga gttgaggcct ggcttgggcg 1260
   ctggggccgc cgcgtgcgaa tctggtggca ccttcgcgcc tgtctcgctg ctttcgataa 1320
   gtctctagcc atttaaaatt tttgatgacc tgctgcgacg ctttttttct ggcaagatag 1380
   tcttgtaaat gcgggccaag atctgcacac tggtatttcg gtttttgggg ccgcgggcgg 1440
   cgacggggcc cgtgcgtccc agcgcacatg ttcggcgagg cggggcctgc gagcgcggcc 1500
   accgagaatc ggacgggggt agtctcaagc tggccggcct gctctggtgc ctggcctcgc 1560
   gccgccgtgt atcgccccgc cctgggcggc aaggctggcc cggtcggcac cagttgcgtg 1620
   agcggaaaga tggccgcttc ccggccctgc tgcagggagc tcaaaatgga ggacgcggcg 1680
   ctcgggagag cgggcgggtg agtcacccac acaaaggaaa agggcctttc cgtcctcagc 1740
   cgtcgcttca tgtgactcca cggagtaccg ggcgccgtcc aggcacctcg attagttctc 1800
   gagcttttgg agtacgtcgt ctttaggttg gggggagggg ttttatgcga tggagtttcc 1860
   ccacactgag tgggtggaga ctgaagttag gccagcttgg cacttgatgt aattctcctt 1920
   ggaatttgcc ctttttgagt ttggatcttg gttcattctc aagcctcaga cagtggttca 1980
   aagttttttt cttccatttc aggtgtcgtg agctagcatg tacaggatgc aactcctgtc 2040
   ttgcattgca ctaagtcttg cacttgtcac gaattcgata tcgacagccc acccctctcc 2100
   tagcccaaga tccgccggcc agttcgccat ggttagatct cccccatgcc catcatgccc 2160
   agcacctgag ttcctggggg gaccatcagt cttcctgttc cccccaaaac ccaaggacac 2220
   tctcatgatc tcccggaccc ctgaggtcac gtgcgtggtg gtggacgtga gccaggaaga 2280
   ccccgaggtc cagttcaact ggtacgtgga tggcgtggag gtgcataatg ccaagacaaa 2340
   gccgcgggag gagcagttca acagcacgta ccgtgtggtc agcgtcctca ccgtcctgca 2400
   ccaggactgg ctgaacggca aggagtacaa gtgcaaggtc tccaacaaag gcctcccgtc 2460
   ctccatcgag aaaaccatct ccaaagccaa agggcagccc cgagagccac aggtgtacac 2520
   cctgccccca tcccaggagg agatgaccaa gaaccaggtc agcctgacct gcctggtcaa 2580
   aggcttctac cccagcgaca tcgccgtgga gtgggagagc aatgggcagc cggagaacaa 2640
   ctacaagacc acgcctcccg tgctggactc cgacggctcc ttcttcctct acagcaggct 2700
   aaccgtggac aagagcaggt ggcaggaggg gaatgtcttc tcatgctccg tgatgcatga 2760
   ggctctgcac aaccactaca cacagaagag cctctccctg tctccgggta aatgagtgga 2820
   tccaccggat ctagataact gatcataatc agccatacca catttgtaga ggttttactt 2880
   gctttaaaaa acctcccaca cctccccctg aacctgaaac ataaaatgaa tgcaattgtt 2940
   gttgttaact tgtttattgc agcttataat ggttacaaat aaagcaatag catcacaaat 3000
   ttcacaaata aagcattttt ttcactgcat tctagttgtg gtttgtccaa actcatcaat 3060
   gtatcttatc ctaggacccc aaaagtttgt ctgcgtattc cagggcgggg ctcagttgaa 3120
   tctcccgcag cacctctacc agcaggtccg cggtgggctg gagaaactcg gccgtcccgg 3180
   ggcaggcggt cgtcgggggt ggaggcgcgg cgcccacccc gtgtgccgcg cctggcgtct 3240
   cctctggggg cgacccgtaa atggttgcag tgatgtaaat ggtgtccgcg gtccagacca 3300
   cggtcaaaat gccggccgtg gcgctccggg cgctttcgcc gcgcgaggag ctgacccagg 3360
   agtcgaacgg atacgcgtac atatgggcgt cccacccgcg ttcgagcttc tggttgctgt 3420
   cccggcctat aaagcggtag gcacaaaatt cggcgcgaca gtcgataatc accaacagcc 3480
   caatgggggt gtgctggata acaacgcctc cgcgcggcag gcggtcctgg cgctcccggc 3540
   cccgtaccat gatcgcgcgg gtgccgtact caaaaacatg caccacctgc gcggcgtcgg 3600
   gcagtgcgct ggtcagcgag gccctggcgt ggcataggct atacgcgatg gtcgtctgtg 3660
   gattggacat 3670
<210> 9
   <211> 6903
   <212> DNA
   <213> Herpes Simplex Virus
<400> 9
   ttagaggcgc cgggagtggg gtcgtcggcg tctgcttttt gtggcggcgt cccgtcgcgg 60
   ggtggggtcc gacgtggcga tgatgggcgg cggcgtggtg aggggcttcg gctgcaggcc 120
   cgcttcatcg tccggcggca gaaccggggt ccgtccagac gttccgttgg taggtcccaa 180
   atcctgtcgc cctacacagc ggcgggtgcg cgaatagtca aagttcacac acccagcctt 240
   cacaggtgtg tggctggcgg cctgcttgcg actgtccagc gcctggcgta tctctttata 300
   aagggccaag cgcgtttctg tttcctgggt gttggcagga aacgcggggt aactcaagtc 360
   ctccaaaaaa cccgccacaa ataaaaaggg gttaacccaa tatgccttct gggcatgcct 420
   atcccacaag accgtgtcca atccgggaca gtgataacgc aaaaatatac cgtctatcaa 480
   agcatagttt atagccgagg gggtctcgta acgccaatca agatcgtcag acgggagcgg 540
   cacacaaggc acctttaata tatcccccac ctctcgagcc acccgactcc gaataacata 600
   ttcggttgaa gacaagcccc cccgcacaca caaaacccca acggcaataa gcccgaccca 660
   acccaaaatc cccatagcgc ctagggtcgg cacccacaga aacctacagt ccccaagtgt 720
   ttgcccagta acacaaccac gacgtcgtgc cacacaagcc ccgtatcccc gttcccgcgc 780
   ttttcgttgg tttatataca cattgattat tgactagtta ttaatagtaa tcaattacgg 840
   ggtcattagt tcatagccca tatatggagt tccgcgttac ataacttacg gtaaatggcc 900
   cgcctggctg accgcccaac gacccccgcc cattgacgtc aataatgacg tatgttccca 960
   tagtaacgcc aatagggact ttccattgac gtcaatgggt ggagtattta cggtaaactg 1020
   cccacttggc agtacatcaa gtgtatcata tgccaagtac gccccctatt gacgtcaatg 1080
   acggtaaatg gcccgcctgg cattatgccc agtacatgac cttatgggac tttcctactt 1140
   ggcagtacat ctacgtatta gtcatcgcta ttaccatggt gatgcggttt tggcagtaca 1200
   tcaatgggcg tggatagcgg tttgactcac ggggatttcc aagtctccac cccattgacg 1260
   tcaatgggag tttgttttgg caccaaaatc aacgggactt tccaaaatgt cgtaacaact 1320
   ccgccccatt gacgcaaatg ggcggtaggc gtgtacggtg ggaggtctat ataagcagag 1380
   ctctctggct aactagagaa cccactgctt actggcttat cgaaattaat acgactcact 1440
   atagggagac ccaagctggc tagcgtttaa acttaagctt ggtaccgagc tcggatccac 1500
   tagtccagtg tggtggaatt cgccaccatg tgccatcagc agctggtcat ctcatggttc 1560
   tccctggtgt ttctggcctc acctctggtc gcaatctggg aactgaaaaa ggatgtgtac 1620
   gtggtggagc tggactggta tcccgatgcc cctggcgaga tggtggtgct gacctgcgac 1680
   acacccgagg aggatggcat cacctggaca ctggatcaga gctccgaggt gctgggaagc 1740
   ggcaagaccc tgacaatcca ggtgaaggag ttcggcgacg ccggccagta cacctgtcac 1800
   aagggaggag aggtgctgag ccactccctg ctgctgctgc acaagaagga ggatggcatc 1860
   tggtccacag acatcctgaa ggatcagaag gagccaaaga acaagacctt cctgcggtgc 1920
   gaggccaaga attatagcgg ccggttcacc tgttggtggc tgaccacaat ctccaccgat 1980
   ctgacatttt ctgtgaagtc tagcagggga tcctctgacc cacagggagt gacatgcgga 2040
   gcagccaccc tgagcgccga gagggtgcgc ggcgataaca aggagtacga gtattccgtg 2100
   gagtgccagg aggactctgc ctgtccagca gcagaggagt ccctgcctat cgaagtgatg 2160
   gtggatgccg tgcacaagct gaagtacgag aattatacca gctccttctt tatccgggac 2220
   atcatcaagc ccgatccccc taagaacctg cagctgaagc ctctgaagaa tagcagacag 2280
   gtggaggtgt cctgggagta ccctgacacc tggagcacac cacactccta tttctctctg 2340
   accttttgcg tgcaggtgca gggcaagtcc aagcgggaga agaaggacag agtgttcacc 2400
   gataagacat ctgccaccgt gatctgtaga aagaacgcct ctatcagcgt gagggcccag 2460
   gaccgctact attctagctc ctggtccgag tgggcctctg tgccttgcag cggcggagga 2520
   ggaggaggat ctatgtgccc agcaaggagc ctgctgctgg tggccacact ggtgctgctg 2580
   gatcacctgt ccctggcaag gaatctgcca gtggcaaccc ctgacccagg catgttcccc 2640
   tgcctgcacc acagccagaa cctgctgagg gccgtgtcca atatgctgca gaaggcccgc 2700
   cagacactgg agttttaccc ttgtaccagc gaggagatcg accacgagga catcacaaag 2760
   gataagacct ccacagtgga ggcctgcctg ccactggagc tgaccaagaa cgagtcctgt 2820
   ctgaacagcc gggagacaag cttcatcacc aacggctcct gcctggcctc tagaaagaca 2880
   agctttatga tggccctgtg cctgtctagc atctacgagg acctgaagat gtatcaggtg 2940
   gagttcaaga ccatgaacgc caagctgctg atggacccca agaggcagat ctttctggat 3000
   cagaatatgc tggccgtgat cgacgagctg atgcaggccc tgaacttcaa tagcgagaca 3060
   gtgcctcaga agtcctctct ggaggagcca gatttctaca agaccaagat caagctgtgc 3120
   atcctgctgc acgcctttcg gatcagagcc gtgacaatcg accgcgtgat gtcctatctg 3180
   aacgcctctg gaggaggagg aagcggagga ggaggatccg gcggcggcgg ctccggctct 3240
   ggcgccacca acttctccct gctgaagcag gcaggcgacg tggaggagaa tccaggacct 3300
   atgcggatct ctaagcctca cctgagaagc atctccatcc agtgctacct gtgcctgctg 3360
   ctgaacagcc actttctgac agaggccggc atccacgtgt tcatcctggg ctgttttagc 3420
   gccggcctgc caaagaccga ggcaaactgg gtgaatgtga tctctgacct gaagaagatc 3480
   gaggatctga tccagagcat gcacatcgat gccacactgt ataccgagtc tgacgtgcac 3540
   cctagctgca aggtgaccgc catgaagtgt ttcctgctgg agctgcaggt catcagcctg 3600
   gagtccggcg acgcaagcat ccacgataca gtggagaacc tgatcatcct ggccaacaat 3660
   agcctgagct ccaacggcaa tgtgaccgag tccggctgca aggagtgtga ggagctggag 3720
   gagaagaaca tcaaggagtt cctgcagagc tttgtgcaca tcgtgcagat gttcatcaat 3780
   acatccggag gaggaggatc aggcggagga ggaagcggcg gcggcggctc tggcagcggc 3840
   gccaccaact tttccctgct gaagcaggcc ggcgatgtgg aagaaaatcc aggaccaatg 3900
   gcaccaagga gagcaagggg atgcagaacc ctgggcctgc ctgccctgtt attactgctg 3960
   ctgctgaggc caccagcaac aaggggaatc acctgtcctc cacccatgag cgtggagcac 4020
   gccgacatct gggtgaagtc ctactctctg tattctaggg agcggtacat ctgcaacagc 4080
   ggctttaaga ggaaggccgg cacatctagc ctgaccgagt gcgtgctgaa caaggccacc 4140
   aatgtggccc actggaccac accttccctg aagtgcatca gggatccagc cctggtgcac 4200
   cagcgccccg cacctccaag cacagtgacc acagcaggag tgacccctca gccagagagc 4260
   ctgtcccctt ctggcaagga gccagcagca tcctctccaa gctccaacaa tacagcagca 4320
   accacagcag caatcgtgcc aggctcccag ctgatgccaa gcaagtcccc ctctaccggc 4380
   accacagaga tctctagcca cgagtcctct cacggcacac caagccagac cacagccaag 4440
   aattgggagc tgaccgcaag cgcctcccac cagccacctg gcgtgtaccc acagggacac 4500
   tccgacacca cagtggccat ctctaccagc acagtgctgc tgtgcggcct gtctgccgtg 4560
   agcctgctgg cctgttatct gaagtccagg cagaccccac cactggcatc tgtggagatg 4620
   gaggccatgg aggccctgcc cgtcacatgg gggactagta gtagagacga ggatctggaa 4680
   aactgtagtc accatctgta agatatccat cacactggcg gccgctcgag catgcatcta 4740
   gagggcccta ttctatagtg tcacctaaat gctagagctc gctgatcagc ctcgactgtg 4800
   ccttctagtt gccagccatc tgttgtttgc ccctcccccg tgccttcctt gaccctggaa 4860
   ggtgccactc ccactgtcct ttcctaataa aatgaggaaa ttgcatcgca ttgtctgagt 4920
   aggtgtcatt ctattctggg gggtggggtg gggcaggaca gcaaggggga ggattgggaa 4980
   gacaatagca ggcatgctgg ggatgcggtg ggctctatgg ggatccacta gttctagagc 5040
   ggccgccgca cataaaggcc cggcgcgacc gacgcccgca gacggcgccg gccacgaacg 5100
   acgggagcgg ctgcggagca cgcggaccgg gagcgggact cgcagagggc cgtcggagcg 5160
   gacggcgtcg gcatcgcgac gccccggctc gggatcggga tcgcatcgga aagggacacg 5220
   cggaaagacc cacccacccc acccacgaaa cacaggggac gcaccccggg ggcctccgac 5280
   gacagaaacc caccggtccg cctttgtgca cgggtaagca ccttgggtgg gcggaggagg 5340
   ggggacgcgg gggcggagga gggggctcac ccgcgttcgt gccttcccgc aggaggaacg 5400
   tcctcgtcga ggcgaccggc ggcgaccgtt gcgtggaccg cttcctgctc gtcggggcga 5460
   ccggcggcga ccgttgcgtg gaccgcttcc tgctcgtcgg gcgggggagc atgtcgtggg 5520
   ccctggaaat ggcggacacc ttcctggaca acatgcgggt tgggcccagg acgtacgccg 5580
   acgtacgcga tgagatcaat aaaagggggc gtgaggaccg ggaggcggcc aaaaccgccg 5640
   tgcacgaccc ggagcgtcct ctgctgcgct ctcccgggct gctgcccaaa atcgccccca 5700
   acgcatcctt gggtgtggca catcgaagaa ccggcgggac cgtgaccgac agtccccgta 5760
   atccggtaac ccgttgagtc ccgggtacga ccatcgccca agtttctggg cggagggtgg 5820
   ttccccccgt ggctctcgag atgagccaga cccaaccccc ggccccagtt gggccgggcg 5880
   acccagatgt ttacttaaaa ggcgtgccgt ccgccggcat gcaccccaga ggtgttcacg 5940
   cacctcgagg acacccgcgc atgatctccg gacccccgca acggggtgat aatgatcaag 6000
   cggcggggca atgtggagat tcgggtctac tacgagtcgg tgcggacact acgatctcga 6060
   agccatctga agccgtccga ccgccaacaa tccccaggac accgcgtgtt ccccgggagc 6120
   cccgggttcc gcgaccaccc cgagaaccta gggaacccag agtaccgcga gctcccagag 6180
   accccagggt accgcgtgac cccagggatc cacgacaacc ccgggagccc cggcctcccc 6240
   gggagccccg gaccccacgc accccccgcg aaccacgtac ggctcgcggg tctgtatagc 6300
   ccgggcaagt atgcccccct ggcgagccca gaccccttct ccccacaaga tggagcgtac 6360
   gctcgggccc gcgtagggct ccacaccgcg gttcgcgtcc cgcccaccgg aagcccaacc 6420
   cacacgcact tgcggcatga cccgggcgat gagccaacct cggatgactc agggctctac 6480
   cctctggacg cccgggcgct tgcgcacctg gtgatgttgc ccgcggacca ccgggccttc 6540
   tttcgaaccg tggtcgaggt gtctcgcatg tgcgctgcaa acgtgcgcga tcccccgccc 6600
   ccggctacag gggccatgtt gggccgccac gcgcggctgg tccacaccca gtggctccgg 6660
   gccaaccaag agacgtcgcc cctgtggccc tggcggacgg cggccattaa ctttatcacc 6720
   accatggccc cccgcgtcca aacccaccga cacatgcacg acctgttgat ggcctgtgct 6780
   ttctggtgct gtctgacaca cgcatcgacg tgttcgtacg cggggctgta ctcgacccac 6840
   tgcctgcatc tgtttggtgc gtttgggtgt ggggacccgg ccctaacccc acccctgtgc 6900
   tag 6903
<210> 10
   <211> 10
   <212> **DNA**
   <213> Homo sapiens
<400> 10
   ggggatttcc 10
<210> 11
   <211> 10
   <212> **DNA**
   <213> Homo sapiens
<400> 11
   gggagattcc 10
<210> 12
   <211> 10
   <212> **DNA**
   <213> Homo sapiens
<400> 12
   gggaatctcc 10
<210> 13
   <211> 10
   <212> **DNA**
   <213> Homo sapiens
<400> 13
   gggagattcc 10
<210> 14
   <211> 11
   <212> **DNA**
   <213> Homo sapiens
<400> **14**
   ggggattccc c 11
<210> 15
   <211> 10
   <212> DNA
   <213> Homo sapiens
<400> 15
   ggggaatccc 10
<210> 16
   <211> 10
   <212> DNA
   <213> Homo sapiens
<400> 16
   gggaatcccc 10
<210> 17
   <211> 10
   <212> DNA
   <213> Homo sapiens
<400> 17
   ggggaaggcc 10
<210> 18
   <211> 10
   <212> DNA
   <213> Homo sapiens
<400> 18
   gggaatttcc 10
<210> 19
   <211> 10
   <212> DNA
   <213> Homo sapiens
<400> 19
   gggaaattcc 10
<210> 20
   <211> 10
   <212> DNA
   <213> Homo sapiens
<400> 20
   gggaactacc 10
<210> 21
   <211> 10
   <212> DNA
   <213> Homo sapiens
<400> 21
   gggattttcc 10
<210> 22
   <211> 10
   <212> DNA
   <213> Homo sapiens
<400> 22
   gggatttcac 10
<210> 23
   <211> 10
   <212> DNA
   <213> Homo sapiens
<400> 23
   ggggctttcc 10
<210> 24
   <211> 10
   <212> DNA
   <213> Homo sapiens
<400> 24
   gggaaagccc 10
<210> 25
   <211> 10
   <212> DNA
   <213> Homo sapiens
<400> 25
   gggaattcac 10
<210> 26
   <211> 10
   <212> DNA
   <213> Homo sapiens
<400> 26
   gggggcttcc 10
<210> 27
   <211> 10
   <212> DNA
   <213> Homo sapiens
<400> 27
   ggggcttccc 10
<210> 28
   <211> 10
   <212> DNA
   <213> Homo sapiens
<400> 28
   gggaagcccc 10
<210> 29
   <211> 10
   <212> DNA
   <213> Homo sapiens
<400> 29
   ggggaagccc 10
<210> 30
   <211> 10
   <212> DNA
   <213> Homo sapiens
<400> 30
   gggaaatccc 10
<210> 31
   <211> 10
   <212> DNA
   <213> Homo sapiens
<400> 31
   gggactttcc 10
<210> 32
   <211> 10
   <212> DNA
   <213> Homo sapiens
<400> 32
   ggggctttcc 10
<210> 33
   <211> 10
   <212> DNA
   <213> Homo sapiens
<400> 33
   ggggaatccc 10
<210> 34
   <211> 10
   <212> DNA
   <213> Homo sapiens
<400> 34
   gggattcccc 10
<210> 35
   <211> 15
   <212> DNA
   <213> Homo sapiens
<400> 35
   ctttgttatg catct 15
<210> 36
   <211> 15
   <212> DNA
   <213> Homo sapiens
<400> 36
   cattgtgatg catat 15
<210> 37
   <211> 15
   <212> DNA
   <213> Homo sapiens
<400> 37
   cattgtaatg caaaa 15
<210> 38
   <211> 15
   <212> DNA
   <213> Homo sapiens
<400> 38
   cattgttatg ctagt 15
<210> 39
   <211> 15
   <212> DNA
   <213> Homo sapiens
<400> 39
   cattgttatg ataaa 15
<210> 40
   <211> 18
   <212> DNA
   <213> Homo sapiens
<400> 40
   ctttgtttgg atgctaat 18

## Description

### CROSS-REFERENCE TO RELATED APPLICATION

This patent application claims the benefit under 35 U.S.C. § 119(e) of U.S. Provisional Patent Application No. 62/329,877 filed April 29, 2016.

### FIELD OF THE INVENTION

This patent application relates generally to enhancing replication of HSV in cancer cells.

### BACKGROUND OF THE INVENTION

Oncolytic viruses (OVs) have been a therapeutic arsenal to specifically destroy cancer cells through oncolysis, which is a killing mechanism characterized by cancer cell lysis through the course of virus lytic replication. In addition to the direct cell killing by the virus. Among the various OVs, herpes simplex virus type 1 ("HSV-1") based OVs are the farthest advanced, e.g., a herpes virus-based OV (T-Vec) has been approved by the U.S. FDA for the treatment of melanoma. Representative examples of HSV vectors include those described in US Patent Nos. 7,223,593, 7,537,924, 7,063,835, 7,063,851, 7,118,755, 8,277,818, and 8,680,068.

The present invention overcomes shortcomings of current commercial oncolytic viruses, and further provides additional unexpected benefits.

### SUMMARY

The invention is defined in the appended claims. Any subject-matter which is described herein, but which is not claimed, does not form part of the invention. Briefly stated, the present invention provides HSV vectors with enhanced replication in cancer cells. Within one embodiment HSV vectors are provided comprising an NF-κB response element in a regulatory region of a viral gene that affects viral replication efficiency. Other claims are directed to an HSV vector comprising an Oct3/4-SOX2response element in a regulatory region of viral gene that affects viral replication efficiency. The viral genes that may have one or both of these response elements include US11 and genes that encode ICP4, ICP27 and ICP8.

Other claims are directed to a vector, wherein the NF-κB response element comprises from 1-15 tandem sequences of GGGAATTTCC (SEQ ID NO: 1) or variants thereof, wherein the variant has at least one nucleotide difference from this sequence. The tandem sequences may be identical or a mix of identical and different sequences or all different sequences. Other claims are directed to a vector, wherein the OCT3/4-SOX2 response element comprises CTACAGAGGTGCATATTAACAGAGCTTTTGTCCTGGAGA (SEQ ID NO: 2) or a variant thereof, wherein the variant has at least 90% identical nucleotides.

In other claims, the HSV vector may further comprise a sequence encoding a therapeutic substance for cancer treatment. The therapeutic substance is IL12, IL15, OX40L, PDL-1 blocker or a PD-1 blocker. One example of suitable blocker is described in United States Patent Application 15/374,893, filed on Dec 09, 2016, hereby incorporated in its entirety.

Other claims are directed to a method of treating cancer, comprising administering an HSV vector as described herein to a patient with cancer or suspected of having cancer. In other claims, a method of treating cancer stem cells and treatment-resistant cancer is provided, comprising administering an HSV vector as described herein to a patient with a treatment resistant cancer or a cancer having cancer stem cells. In other claims, the cancers are colon cancer, lung cancer, breast cancer, prostate cancer, brain cancer, or bladder cancer.

The SUMMARY has been provided to introduce certain concepts in a simplified form that are further described in detail below in the DETAILED DESCRIPTION OF THE INVENTION. Except where otherwise expressly stated, this SUMMARY is not intended to identify key or essential features of the claimed subject matter, nor is it intended to limit the scope of the claimed subject matter.

The details of one or more embodiments are set forth in the description below. The features illustrated or described in connection with one exemplary embodiment may be combined with the features of other embodiments. Thus, any of the various embodiments described herein can be combined to provide further embodiments. Aspects of the embodiments can be modified, if necessary to employ concepts of the various patents, applications and publications as identified herein to provide yet further embodiments. Other features, objects and advantages will be apparent from the description, the drawings, and the claims.

### BRIEF DESCRIPTION OF THE DRAWINGS

Exemplary features of the present disclosure, its nature and various advantages will be apparent from the accompanying drawings and the following detailed description of various embodiments. Non-limiting and non-exhaustive embodiments are described with reference to the accompanying drawings, wherein like labels or reference numbers refer to like parts throughout the various views unless otherwise specified. The sizes and relative positions of elements in the drawings are not necessarily drawn to scale. For example, the shapes of various elements are selected, enlarged, and positioned to improve drawing legibility. The particular shapes of the elements as drawn have been selected for ease of recognition in the drawings. One or more embodiments are described hereinafter with reference to the accompanying drawings in which:
Figures 1A-B are schematics of exemplary oHSV vectors.
Figure 2 is a graph showing enhanced viral production of an exemplary oHSV-1 vector carrying a NF-κB response element in the regulatory region of ICP4 in the presence of TNFα.
Figures 3A-B present the schematic and sequence (SEQ ID NO: 3) of OS-ICP27.
Figures 4A-B present the schematic and sequence (SEQ ID NO: 4) of NO-ICP27-145.
Figures 5A-B present the schematic and sequence (SEQ ID NO: 5) of NO-ICP27-99.
Figure 6 shows a schematic of the modified ICP34.5 region (SEQ ID NO: 6) for virus hVG161.
Figure 7 shows a schematic of a modified UL54 promoter region (SEQ ID NO: 7) for virus hVG161.
Figure 8 shows a schematic of hVG161.viral genome with an insertion of a PD-L1 blocker (SEQ ID NO: 8).
Figure 9 shows a schematic of hVG161 modified TR region (SEQ ID NO: 9).
Figures 10A-10K show assay results for a virus comprising mutations in ICP27 promoter/regulatory region.
Figures 11A-11C show ELISA and Western blot data for IL-12 expression following hVG161 infection of cells.
Figures 12A-12C show ELISA and Western blot data for IL-15 expression following hVG161 infection of cells.
Figures 13A-13C show ELISA and Western blot data for IgG4 expression following hVG161 infection of cells.
Figures 14A-14E show results of cell infection with VG161-PLBh and VG161-15h.
Figures 15A-15D show results of in vitro assays for various constructs. Fig. 15A-15B show results of cell transfection with IL-TF-Fc plasmid carrying IL-12, IL-15, and PD-L1 blocker. Figures 15C-15D show results of cell infection with a variety of mutant viruses including hVG161.
Figures 16A-16E show results of cell viability assays for hVG161 and HSV-345 on human tumor cell lines and Vero cell line.
Figures 17A-17J show results of in vitro assays for various constructs. Figures 17A-17E show results of cell viability assays for mVG161 and HSV-345 on mouse tumor cell lines and Vero cell line; figures 17F-17J show the characterization of transgene expression following mVG161 or VG001 infection of CT26 mouse tumor cells.
Figures 18A-18E show results of in vitro characterization of transgene expression following hVG161 or VG001 infection of various cell lines.
Figures 19A-19G show results of assays to evaluate the ability of hVG161 to kill a variety of human cancer cells in vitro.
Figures 20A-20G show results of in vivo assays for mVG161 and hVG161 constructs.
Figures 21A-21C show growth curves for different viruses on three different human cell lines.
Figures 22A-22D show growth curves of mVG161 and HSV-345 on mouse tumor cell lines and Vero cell line.
Figures 23A-23E show growth curves of hVG161 and HSV-345 on human tumor cell lines and Vero cell line.
Figures 24A-24D show growth curves of NO-ICP27 and HSV-345 on various cell lines.

### DETAILED DESCRIPTION OF THE INVENTION

The present invention may be understood more readily by reference to the following detailed description of preferred embodiments of the invention and the Examples included herein. Briefly stated, the present disclosure provides compositions and methods for treating cancer using HSV vectors that comprise sequences that are responsive to NF-κB or Oct-3/4-SOX2 or both transcriptional activators.

Within certain embodiments of the invention, the present disclosure provides methods and compositions, to cancer cells, typically in vivo to a subject in need of cancer treatment. The term "cancer," as used herein, refers to a cancer of any kind and origin, including treatment resistant cancers (e.g., resistant to conventional radiation and/or chemotherapy); cancer stem cells, tumor-forming cells, blood cancers, and transformed cells. The term "cancer cell," as used herein, includes cancer or tumor-forming cells, transformed cells, or a cell that is susceptible to becoming a cancer or tumor-forming cell. Representative forms of cancer include carcinomas, sarcomas, myelomas, leukemia's, lymphomas, and mixed types of the above. Further examples include, but are not limited to those discussed in more detail below.

Some cancer cells, especially cancer stem cells, are relatively resistant to oncolytic HSV-1 virus induced oncolysis. To increase the therapeutic efficacy of oHSV-1, the viral genes that regulate viral replication can be modified in their regulatory region to insert an NFkB response element or an Oct3/4-SOX2response element or both to make the gene expression particularly responsive to activation of NFkB in infected cells. Since NFkB pathway is often upregulated in cancer cells and cancer stem cells, this modification will enhance viral production and oncolytic activity of oHSV for more effective cell killing.

### A. NF-κB and Oct-3/4-SOX2 response elements

Oct-3/4-SOX2 complex (also designated Oct-3, Oct4 and Pou5f1) and NF-κB are expressed in many different tumors. Oct4 and SOX2 are transcriptional factor expressed in stem cells. The two proteins form a complex to bind to the regulatory regions of genes involved in maintaining stem cells and pluripotent cells. In cancer stem cells it maintains a "stemness" state. Cancer stem cells have been identified in a variety of solid malignancies. They are a small population of tumor cells with stem cell characteristics, which are a likely cause of relapse in cancer patients. NF-κB (Nuclear factor-kappaB) is a family of transcription factors. In essentially all unstimulated nucleated cells, NPκB complexes are bound to an inhibitor of NP_{K}B (IκB) proteins. When released from the inhibitor, NF-κB translocates into the nucleus and targets a sequence in enhancer elements, activating or increasing gene transcription. NF-κB is activated in many cancer cell types, including hepatocellular carcinoma, lymphomas, leukemias, colon cancer, lung cancer, breast cancer, prostate cancer, brain cancer, and bladder cancer. It is also activated in cancer stem cells.

Response elements for NF_{K}B includes the decameric binding sequence. The most common sequence is GGGAATTTCC (SEQ ID NO: 1), although variants can be targets as well. In general, a variant will have one or two differences to the most common sequence.

Some known NF_{K}B binding sequence variants include:

**TABLE 1**

| SEQUENCE | SEQ ID NO. |
|---|---|
| GGGGATTTCC | 10 |
| GGGAGATTCC | 11 |
| GGGAATCTCC | 12 |
| GGGAGATTCC | 13 |
| GGGGATTCCCC | 14 |
| GGGGAATCCC | 15 |
| GGGAATCCCC | 16 |
| GGGGAAGGCC | 17 |
| GGGAATTTCC | 18 |
| GGGAAATTCC | 19 |
| GGGAACTACC | 20 |
| GGGA TTTTCC | 21 |
| GGGA TTTCAC | 22 |
| GGGGCTTTCC | 23 |
| GGGAAAGCCC | 24 |
| GGGAATTCAC | 25 |
| GGGGGCTTCC | 26 |
| GGGGCTTCCC | 27 |
| GGGAAGCCCC | 28 |
| GGGGAAGCCC | 29 |
| GGGAAATCCC | 30 |
| GGGACTTTCC | 31 |
| GGGGCTTTCC | 32 |
| GGGGAATCCC | 33 |
| GGGA TTCCCC | 34 |

The NF-κB response element comprises at least one of the binding sequences and may comprise from 2 up to 15 binding sequences in tandem array. When more than one sequence is present, the more than one sequence may be identical or different. For example, a response element comprising five binding sequences may have all five sequences identical, or four identical and one different, or three identical and two different, or two identical and three different, or five different sequences. As well, the tandem response elements may have nucleotides in between the elements. There may be from one to about ten additional nucleotides.

Oct-3/4 binds an octameric sequence ATTTGCAT. Sox2 binds to an octameric sequence CTTTTGTC. It has been found that Oct4/Sox2 heterodimer binds to CTACAGAGGTGCATATTAACAGAGCTTTTGTCC-TGGAGA [SEQ ID No. 2] or variants of the sequence. (Wang et al. J Biol Chem 282: 12822, 2007). Variants will generally be at least 90% identical to this sequence, or at least 95% identical, or at least 98% identical.

The Oct-3/4-Sox2 response element comprises at least one of the binding sequences and may comprise from 2 up to 15 binding sequences in tandem array. When more than one sequence is present, the more than one sequence may be identical or different. For example, a response element comprising five binding sequences may have all five sequences identical, or four identical and one different, or three identical and two different, or two identical and three different, or five different sequences. As well, the tandem response elements may have nucleotides in between the elements. There may be from one to about ten additional nucleotides.

Some possible variants of the sequence for binding the Oct4/Sox2 complex can be found in MOLECULAR AND CELLULAR BIOLOGY (July 2005, p. 6031-6046), and include:

| | |
|---|---|
| CTTTGTTATGCATCT | SEQ ID NO. 35 |
| CATTGTGATGCATAT | SEQ ID NO. 36 |
| CATTGTAATGCAAAA | SEQ ID NO. 37 |
| CATTGTTATGCTAGT | SEQ ID NO. 38 |
| CATTGTTATGATAAA | SEQ ID NO. 39 |
| CTTTGTTTGGATGCTAAT | SEQ ID NO. 40 |

The vectors may contain a mixture of NF-κB and Oct-3/4-Sox 2 response elements. When each is present, generally the response elements will be in tandem array, such as seen in the constructs of Example 2. The order of the response elements may be with either element in the 5' position. It is also contemplated that there can be more than one of NF-κB or more than one of Oct-3/4-Sox 2 response elements or more than one of each response elements. In the case where there are multiple of one or more of the elements, they may be in any order. As well, there may be additional nucleotides between the response elements.

### B. HSV vector constructs

An oncolytic virus is a virus that will lyse cancer cells (oncolysis), preferably in a selective manner. Viruses that selectively replicate in dividing cells over non-dividing cells are often oncolytic.

Herpes Simplex Virus (HSV) 1 and 2 are members of the Herpesviridae family, which infect humans. The HSV genome contains two unique regions, which are designated unique long (UL) and unique short (US) region. Each of these regions is flanked by a pair of inverted terminal repeat sequences. There are about 75 known open reading frames. The viral genome has been engineered to develop oncolytic viruses for use in e.g. cancer therapy. Tumor-selective replication of HSV is conferred by mutation of the HSV ICP34.5 (also called γ34.5) gene. HSV contains two copies of ICP34.5. Mutants inactivating one or both copies of the ICP34.5 gene are known to lack neurovirulence, i.e. be avirulent/ non-neurovirulent and be oncolytic.

Suitable oncolytic HSV may be derived from either HSV-1 or HSV-2, including any laboratory strain or clinical isolate. In some embodiments, the oHSV may be or may be derived from one of laboratory strains HSV-1 strain 17, HSV-1 strain F, or HSV-2 strain HG52. In other embodiments, it may be of or derived from non-laboratory strain JS-1. Other suitable HSV-1 viruses are in the table 2 below.

**TABLE 2**

| Virus | Name | references |
|---|---|---|
| HSV-1 | HrrR3 | [1] |
| | G207 | [2, 3] |
| | G47Delta | [4] |
| | HSV 1716 | [5, 6] |
| | HF10 | [7] |
| | NV1020 | [8] |
| | T-VEC | [9] |
| | J100 | [10] |
| | M002 | [11] |
| | NV1042 | [12] |
| | G207-IL2 | [13] |
| | rQNestin34.5 | [14] |
| | G47Δ-mIL-18 | [15] |

In some embodiments, the oHSV has one or both of the γ34.5 genes are modified such that it is incapable of expressing a functional ICP34.5 protein. The genes may be modified by mutation of one or more nucleotides, insertions, deletions, substitutions, etc. The alteration may be in the coding sequence, non-coding sequence (e.g., promoter) or both. In some embodiments, both copies of the γ34.5 genes are mutated.

The oHSV may have additional mutations, which may include disabling mutations e.g., deletions, substitutions, insertions), which may affect the virulence of the virus or its ability to replicate. For example, mutations may be made in any one or more of ICP6, ICPO, ICP4, ICP27, ICP47, ICP 24, ICP56. Preferably, a mutation in one of these genes (optionally in both copies of the gene where appropriate) leads to an inability (or reduction of the ability) of the HSV to express the corresponding functional polypeptide. In some embodiments, the promoter of a viral gene may be substituted with a promoter that is selectively active in target cells or inducible.

The oHSV may also have genes and nucleotide sequences that are non-HSV in origin. For example, a sequence that encodes a prodrug, a sequence that encodes a cytokine or other immune stimulating factor, a tumor-specific promoter, an inducible promoter, an enhancer, a sequence homologous to a host cell, among others may be in the oHSV genome. Exemplary sequences encode IL12, IL15, OX40L, PDL-1 blocker or a PD-1 blocker.

The regulatory region of viral genes encoding US11, ICP4, ICP27, and ICP8 may be modified to comprise a response element for NF-κB or Oct-3/4-SOX2 or both NF-κB and Oct-3/4-SOX2. (ICP4 is encoded by RS1; US11 is encoded by US11; ICP27 is encoded by UL54; ICP8 is encoded by UL29. The HSV-1 vector can have one viral gene that contains one or both response elements. Alternatively, the vector can have more than one viral gene that contains one or both response elements. In general, the response element will be in the viral gene enhancer region. The response element can replace or be in addition to the viral enhancer. The viral promoter may be replaced, generally with a tumor-specific promoter, such as survivin promoter. Other tumor-specific promoters are known in the art. Other gene elements may be modified as well. For example, the 5' UTR of the viral gene may be replaced with an exogenous UTR.

### C. Therapeutic compositions

Therapeutic compositions are provided that may be used to prevent, treat, or ameliorate the effects of a cancer. More particularly, some therapeutic compositions comprise an oncolytic virus as described herein. Within preferred embodiments, the therapeutic composition can comprise an oncolytic virus as described herein.

In certain embodiments, the compositions will further comprise a pharmaceutically acceptable carrier. The phrase "pharmaceutically acceptable carrier" is meant to encompass any carrier, diluent or excipient that does not interfere with the effectiveness of the biological activity of the oncolytic virus and that is not toxic to the subject to whom it is administered (see generally Remington: The Science and Practice of Pharmacy, Lippincott Williams & Wilkins; 21st ed. (May 1, 2005 and in The United States PharmacopE1A: The National Formulary (USP 40 - NF 35 and Supplements).

In the case of an oncolytic virus as described herein, non-limiting examples of suitable pharmaceutical carriers include phosphate buffered saline solutions, water, emulsions (such as oil / water emulsions), various types of wetting agents, sterile solutions, and others. Additional pharmaceutically acceptable carriers include gels, bioadsorbable matrix materials, implantation elements containing the oncolytic virus, or any other suitable vehicle, delivery or dispensing means or material(s). Such carriers can be formulated by conventional methods and can be administered to the subject at an effective dose. Additional pharmaceutically acceptable excipients include, but are not limited to, water, saline, polyethyleneglycol, hyaluronic acid and ethanol. Pharmaceutically acceptable salts can also be included therein, e.g., mineral acid salts (such as hydrochlorides, hydrobromides, phosphates, sulfates, and the like) and the salts of organic acids (such as acetates, propionates, malonates, benzoates, and the like).. Such pharmaceutically acceptable (pharmaceutical-grade) carriers, diluents and excipients that may be used to deliver the oHSV to a target cancer cell will preferably not induce an immune response in the individual (subject) receiving the composition (and will preferably be administered without undue toxicity).

The compositions provided herein can be provided at a variety of concentrations. For example, dosages of oncolytic virus can be provided which ranges from about 10⁶ to about 10⁹ pfu. Within further embodiments, the dosage form can range from about 10⁶ to about 10⁸ pfu/ml, with up to 4 mls being injected into a patient with large lesions (e.g., >5 cm) and smaller amounts (e.g, up to 0.1mls) in patients with small lesions (e.g., < 0.5 cm) every 2 - 3 weeks, of treatment.

Within certain embodiments of the invention, lower dosages than standard may be utilized. Hence, within certain embodiments less than about 10⁶ pfu/ml (with up to 4 mls being injected into a patient every 2 - 3 weeks) can be administered to a patient.

The compositions may be stored at a temperature conducive to stable shelf-life, and includes room temperature (about 20°C), 4°C, -20°C, - 80°C, and in liquid N2. Because compositions intended for use *in vivo* generally don't have preservatives, storage will generally be at colder temperatures. Compositions may be stored dry (e.g., lyophilized) or in liquid form.

### D. Administration

In addition to the compositions described herein, various methods of using such compositions to treat or ameliorate cancer are provided, comprising the step of administering an effective dose or amount of a HSV vector as described herein to a subject.

The terms "effective dose" and "effective amount" refers to amounts of the oncolytic virus that is sufficient to effect treatment of a targeted cancer, e.g., amounts that are effective to reduce a targeted tumor size or load, or otherwise hinder the growth rate of targeted tumor cells. More particularly, such terms refer to amounts of oncolytic virus that is effective, at the necessary dosages and periods of treatment, to achieve a desired result. For example, in the context of treating a cancer, an effective amount of the compositions described herein is an amount that induces remission, reduces tumor burden, and/or prevents tumor spread or growth of the cancer. Effective amounts may vary according to factors such as the subject's disease state, age, gender, and weight, as well as the pharmaceutical formulation, the route of administration, and the like, but can nevertheless be routinely determined by one skilled in the art.

The therapeutic compositions are administered to a subject diagnosed with cancer or is suspected of having a cancer. Subjects may be human or non-human animals.

The compositions are used to treat cancer. The terms "treat" or "treating" or "treatment," as used herein, means an approach for obtaining beneficial or desired results, including clinical results. Beneficial or desired clinical results can include, but are not limited to, alleviation or amelioration of one or more symptoms or conditions, diminishment of extent of disease, stabilized (i.e. not worsening) state of disease, preventing spread of disease, delay or slowing of disease progression, amelioration or palliation of the disease state, diminishment of the reoccurrence of disease, and remission (whether partial or total), whether detectable or undetectable. The terms "treating" and "treatment" can also mean prolonging survival as compared to expected survival if not receiving treatment.

Representative forms of cancer include carcinomas, leukemia's, lymphomas, myelomas and sarcomas. Further examples include, but are not limited to cancer of the bile duct cancer, brain (e.g., glioblastoma), breast, cervix, colorectal, CNS (e.g., acoustic neuroma, astrocytoma, craniopharyogioma, ependymoma, glioblastoma, hemangioblastoma, medulloblastoma, menangioma, neuroblastoma, oligodendroglioma, pinealoma and retinoblastoma), endometrial lining, hematopoietic cells (e.g., leukemia's and lymphomas), kidney, larynx, lung, liver, oral cavity, ovaries, pancreas, prostate, skin (e.g., melanoma and squamous cell carcinoma) and thyroid. Cancers can comprise solid tumors (e.g., sarcomas such as fibrosarcoma, myxosarcoma, liposarcoma, chondrosarcoma and osteogenic sarcoma), be diffuse (e.g., leukemia's), or some combination of these (e.g., a metastatic cancer having both solid tumors and disseminated or diffuse cancer cells). Cancers can also be resistant to conventional treatment (e.g. conventional chemotherapy and/or radiation therapy).

Benign tumors and other conditions of unwanted cell proliferation may also be treated.

The oHSV as described herein may be given by a route that is e.g. oral, topical, parenteral, systemic, intravenous, intramuscular, intraocular, intrathecal, intratumor, subcutaneous, or transdermal. Within certain embodiments the oncolytic virus may be delivered by a cannula, by a catheter, or by direct injection. The site of administration may be intra-tumor or at a site distant from the tumor. The route of administration will often depend on the type of cancer being targeted.

The optimal or appropriate dosage regimen of the oncolytic virus is readily determinable within the skill of the art, by the attending physician based on patient data, patient observations, and various clinical factors, including for example a subject's size, body surface area, age, gender, and the particular oncolytic virus being administered, the time and route of administration, the type of cancer being treated, the general health of the patient, and other drug therapies to which the patient is being subjected. According to certain embodiments, treatment of a subject using the oncolytic virus described herein may be combined with additional types of therapy, such as chemotherapy using, e.g., a chemotherapeutic agent such as etoposide, ifosfamide, adriamycin, vincristin, doxicyclin, and others.

oHSV may be formulated as medicaments and pharmaceutical compositions for clinical use and may be combined with a pharmaceutically acceptable carrier, diluent, excipient or adjuvant. The formulation will depend, at least in part, on the route of administration. Suitable formulations may comprise the virus and inhibitor in a sterile medium. The formulations can be fluid, gel, paste or solid forms. Formulations may be provided to a subject or medical professional

A therapeutically effective amount is preferably administered. This is an amount that is sufficient to show benefit to the subject. The actual amount administered and time-course of administration will depend at least in part on the nature of the cancer, the condition of the subject, site of delivery, and other factors.

Within yet other embodiments of the invention the oncolytic virus can be administered intratumorally, or, after surgical resection of a tumor.

The following examples are offered by way of illustration, and not by way of limitation.

The following examples are offered by way of illustration, and not by way of limitation.

### EXAMPLES

### EXAMPLE 1

### ENHANCEMENT OF VIRAL PRODUCTION

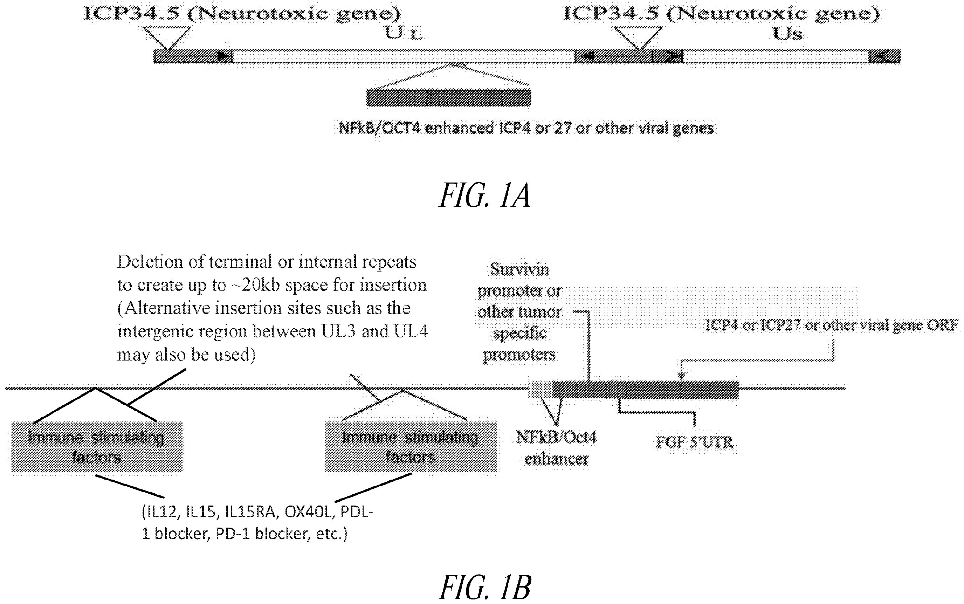

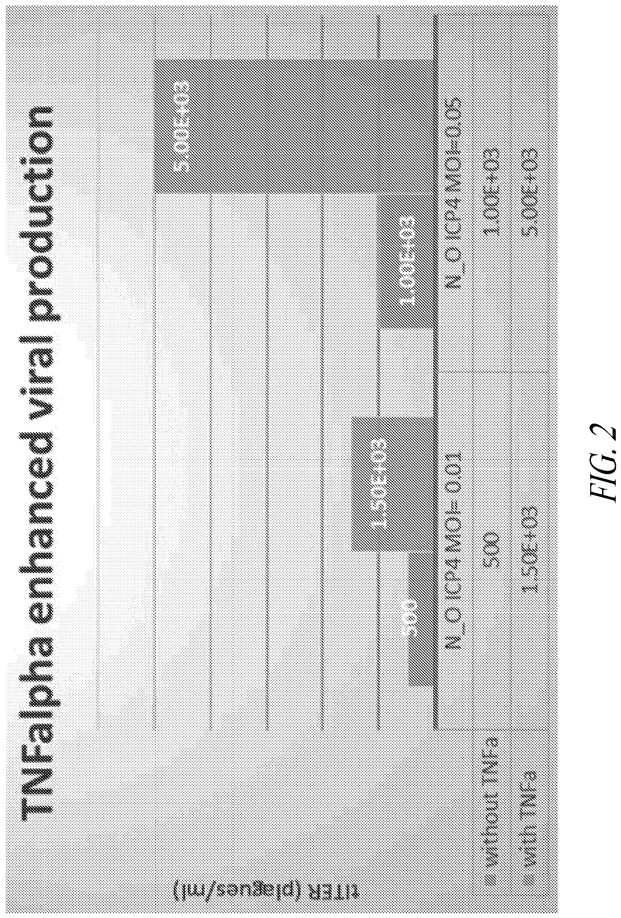

In this example, the production of oHSV-1 shown in Figure 1A was measured in the presence of an activator of NF-κB. The virus has a NF-κB response element in the regulatory region of ICP4. Viral production was significantly enhanced in the presence of TNFalpha that activates NF-κB (Figure 2).

### EXAMPLE 2

### EXEMPLARY CONSTRUCTS

In this example, various constructs and their sequences are presented.

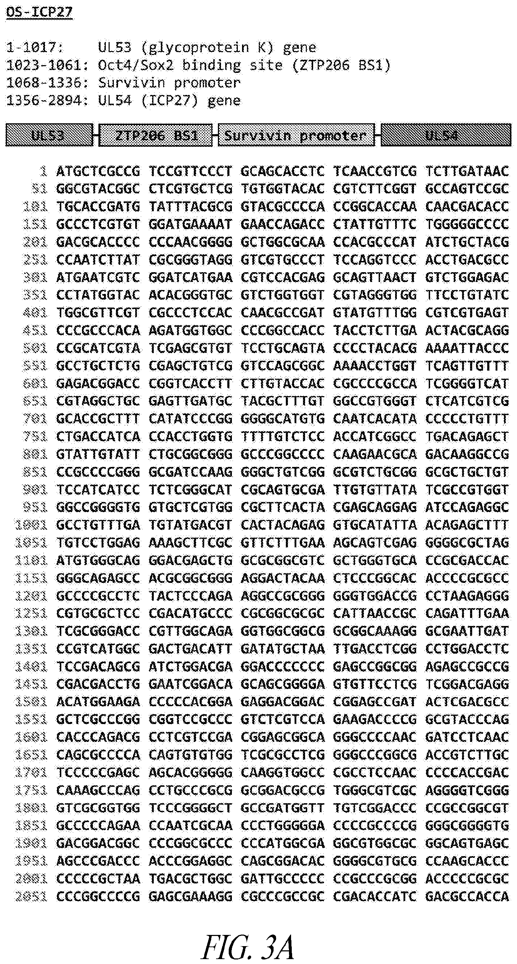

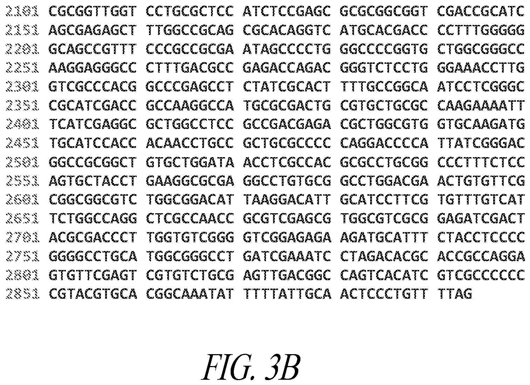

OS-ICP27 comprises ZTP206 BS1 (Oct4/Sox2 binding site) and a survivin promoter which replace the native intergenic region between UL53 (glycoprotein K) and UL54 (ICP27) (Fig. 3).

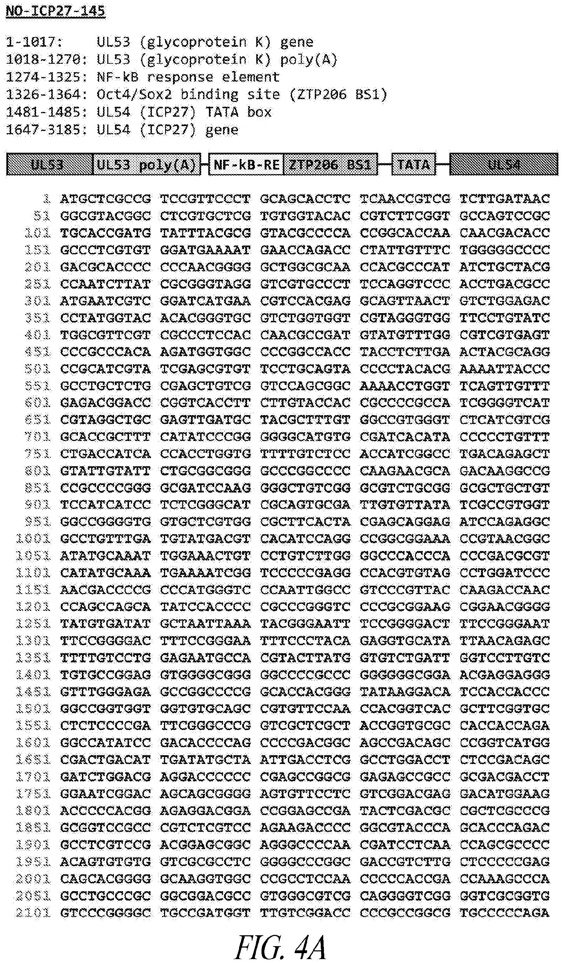

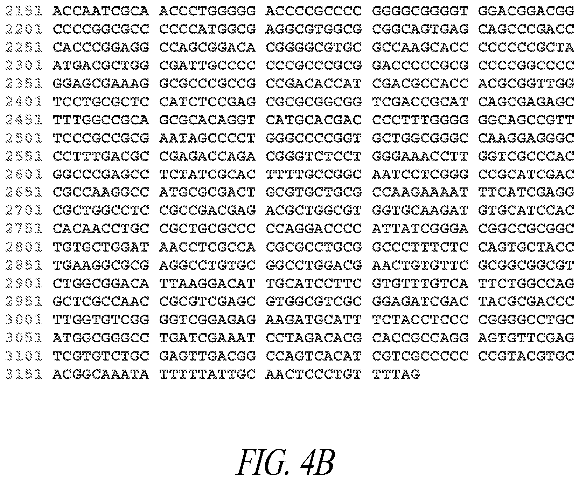

NO-ICP27-145 comprises an NF- kB response element and Oct4/Sox2 binding site (ZTP206 BS1) inserted within the intergenic region between UL53 (glycoprotein K) and UL54 (ICP27) at a location downstream of the UL53 (glycoprotein K) poly(A) and upstream of the UL54 (ICP27) TATA box. (Fig. 4).

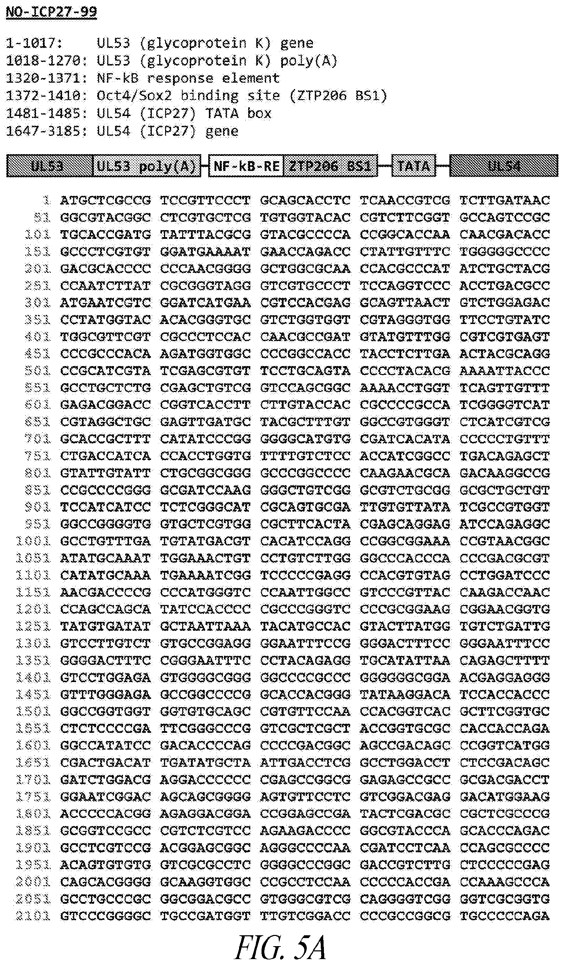

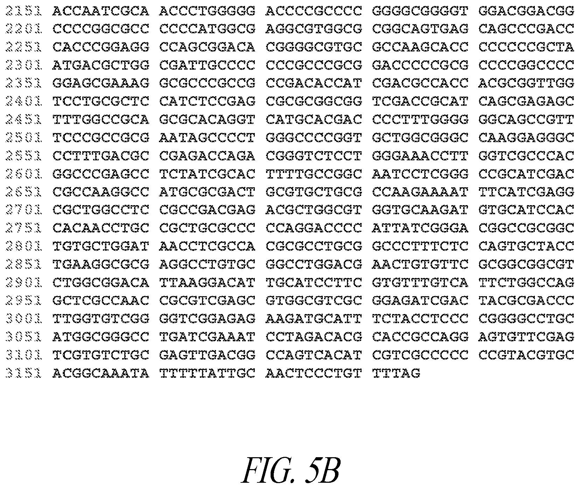

NO-ICP27-99 comprises an NF- kB response element and Oct4/Sox2 binding site (ZTP206 BS1) inserted within the intergenic region between UL53 (glycoprotein K) and UL54 (ICP27) at a location downstream of the UL53 (glycoprotein K) poly(A) and upstream of the UL54 (ICP27) TATA box. (Fig. 5).

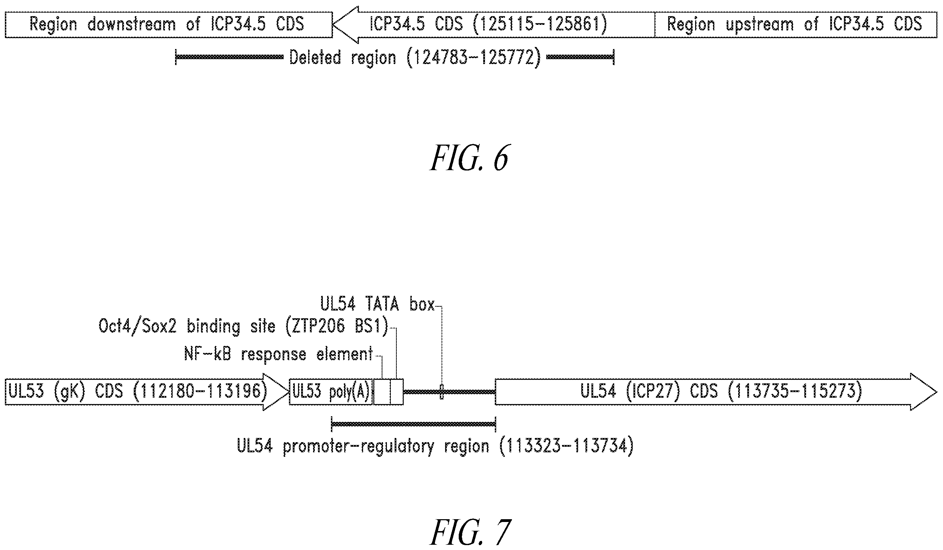

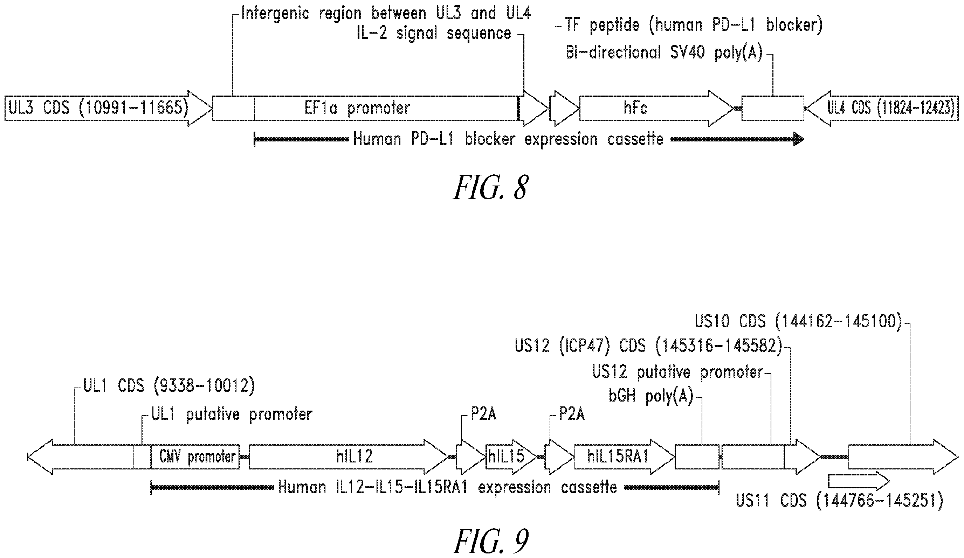

hVG161 comprises a modified ICP34.5 region (Fig. 6; SEQ ID NO. 6), a modified UL54 promoter-regulatory region (Fig. 7; SEQ ID NO. 7), an insertion of a PD-L1 blocker within the intergenic region between UL3 and UL4 (Fig. 8; SEQ ID No. 8), and a modified terminal repeat (TR) region carrying an expression cassette encoding IL-12, IL-15, and IL-15 receptor alpha subunit (Fig. 9; SEQ ID NO. 9). These four viruses also have a modified and partially deleted ICP 34.5 region.

mVG161 is a functionally identical mouse version of hVG161 except that that mVG161 carries a mouse version of IL-12 and a mouse PD-L1 blocker in the same location on the viral genome where hVG161 carries a human IL-12 and a human PD-L1 blocker.

### EXAMPLE 3

### ABBREVIATIONS USED IN SUBSEQUENT EXAMPLES

TF-Fc: PD-L1 blocking peptide (TF) fused to Fc and used for construction of VG161.

IL-TF-Fc: plasmid carrying IL-12, IL-15, and PD-L1 blocker.

HSV-345: ICP34.5-deleted virus.

OS-ICP27 2-11: ICP34.5-deleted virus with Oct4/Sox2 binding site and surviving promoter (OS) inserted within the promoter-regulatory region of ICP27 (OS-ICP27) that was not used for construction of VG161.

OS-ICP27 5-7: ICP34.5-deleted virus with OS-ICP27 mutation that was not used for construction of VG161.

NO-ICP27 1-4-4 (also known as NO-ICP27-145): ICP34.5-deleted virus with NF-kB response element and Oct4/Sox2 binding site (NO) inserted within the promoter-regulatory region of ICP27 (NO-ICP27) at a location 145 bp upstream of the transcription start site of ICP27 and that was used for construction of VG161.

NO-ICP27 5-2-2 (also known as NO-ICP27-99): ICP34.5-deleted virus with NF-kB response element and Oct4/Sox2 binding site (NO) inserted within the promoter-regulatory region of ICP27 (NO-ICP27) at a location 99 bp upstream of the transcription start site of ICP27 and that was not used for construction of VG161.

VG001 (also known as VG160): backbone virus that was used for construction of VG161 (NO-ICP27 1-4-4 mutant carrying an exogenous promoter and poly(A) flanking an empty MCS within deleted terminal repeat region of the viral genome that is subsequently used for insertion of the IL-12/IL-15 expression cassette).

VG001-15h (also known as VG161-15h): VG001 carrying human IL-15.

VG001 -1215h (also known as VG161-1215h): VG001 carrying human IL-12 and human IL-15.

VG001-PLBh (also known as VG161-PLBh): VG001 carrying human PD-L1 blocker inserted within intergenic region between UL3 and UL4.

8-8-15RA1-PDL1b: VG001 carrying human IL-15 and human PD-L1 blocker.

VG161-1215PLBm (also known as mVG161): VG001 carrying mouse IL-12, human IL-15, and mouse PD-L1 blocker.

VG161-1215PLBh (also known as hVG161 or VG161): VG001 carrying human IL-12, human IL-15, and human PD-L1 blocker.

### EXAMPLE 4

### RESULTS OF EXEMPLARY CONSTRUCTS COMPRISING MUTATIONS IN THE ICP27

### PROMOTER/REGULATORY REGION.

In this example, viruses with mutations in the ICP27 promoter-regulatory region were used to infect cells.

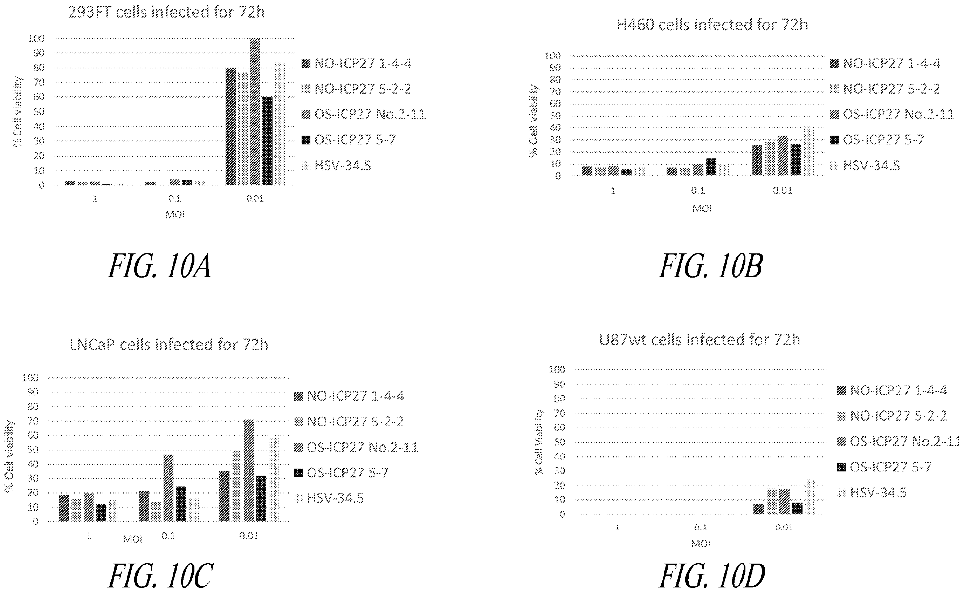

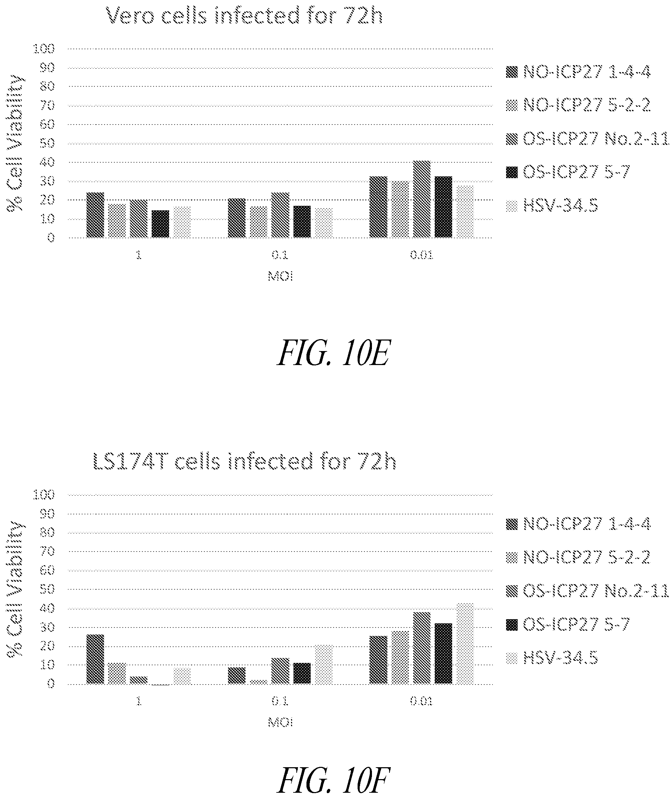

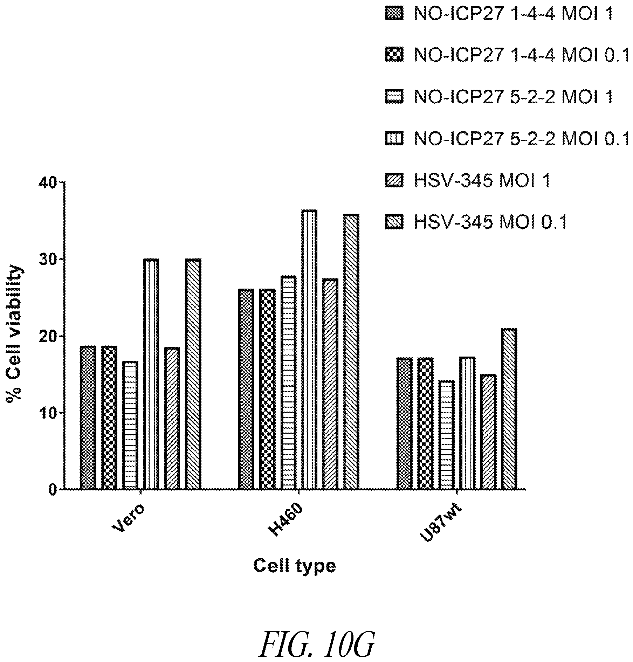

In Fig. 10A-G, Vero, LS174T, 293FT, H460, U87wt, and LNCaP cells were infected with the indicated HSV-1 mutants at MOI 0.01, 0.1, and 1. Cell viability was quantified using MTT assay at 72 hours post infection.

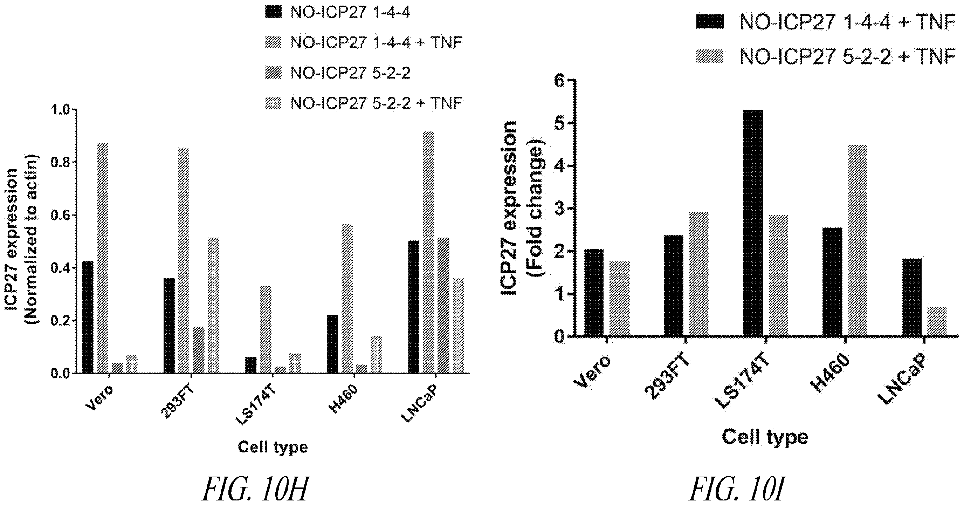

In Fig. 10H-I, Vero, 293FT, LS174T, H460, and LNCaP cells were infected with HSV-1 mutants NO-ICP27 1-4-4 and NO-ICP27 5-2-2 at MOI 1 both with and without induction using 20 ng/mL of TNFα. Infected cells were harvested 5 hours post-infection and the cell lysates were probed with anti-ICP27 and anti-actin antibodies to detect expression of ICP27 and actin, respectively. Actin was used as a loading control for the western blot. Band intensity was quantified and used to estimate expression of ICP27 normalized to actin (Fig. 10H). TNFα-mediated induction of ICP27 expression was measured (Fig. 10I) as fold change in normalized ICP27 expression following treatment with TNFα. A robust response to TNFα was evident in both versions of NO-ICP27 but the NO-ICP27 1-4-4 variant was ultimately selected for inclusion in the VG161 mutant virus due to stronger upregulation of ICP27 expression.

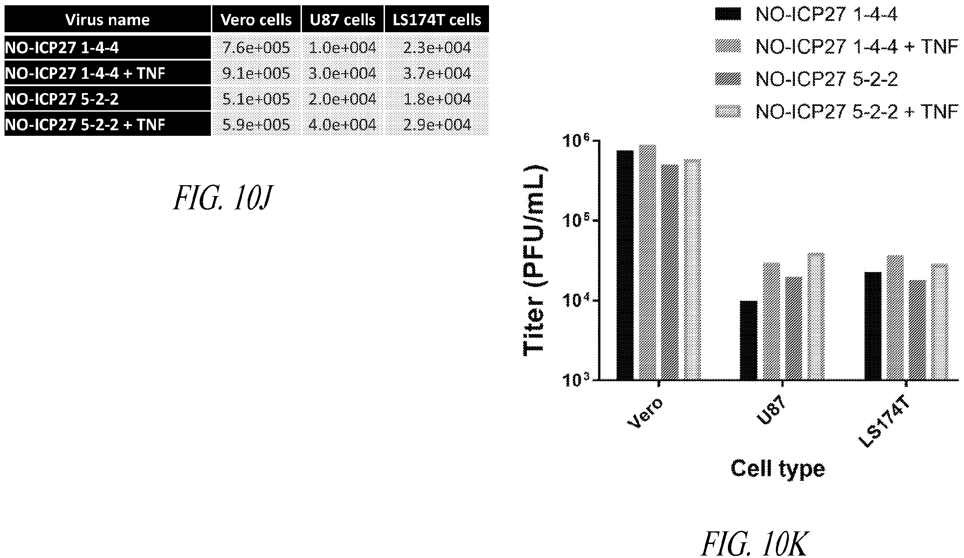

In Fig. 10J-K, Vero, U87, and LS174T cells were infected with HSV-1 mutants NO-ICP27 1-4-4 and NO-ICP27 5-2-2 at MOI 0.1 both with and without induction using 20 ng/mL of TNFα. Cells were harvested 24 hours post-infection and subjected to one freeze/thaw cycle to completely lyse the infected cells prior to titration on Vero cells. Figure 10J shows the raw titer data while Figure 10K is a graphical representation of the data from Figure 10J. Both NO-ICP27 mutants exhibit enhanced replication on Vero, U87, and LS174T cells after TNFα induction. The NO-ICP27 1-4-4 mutant was ultimately selected as a backbone for insertion of the PD-L1 blocker and the IL-12/IL-15 expression cassette to create the final VG161 mutant virus.

### EXAMPLE 5

### EXPRESSION OF IL-12 FOLLOWING INFECTION OF CELLS BY HVG161

In this Example, Western blot and ELISA data of Il-12 expression is shown.

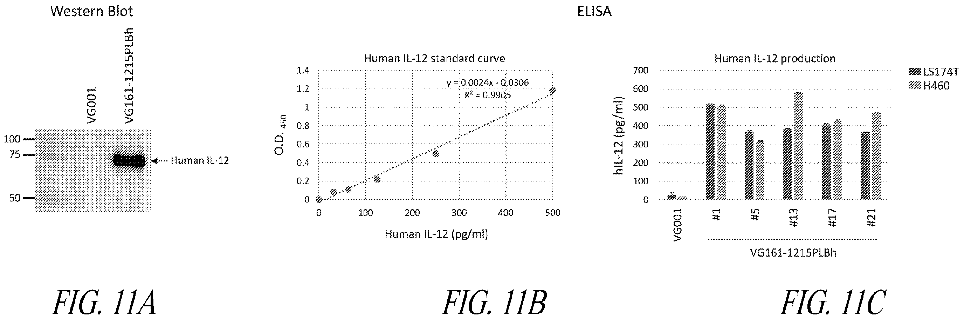

Fig.11A shows Western Blot results after VG161-1215PLBh virus infection. H460 tumour cells were infected with VG161-1215PLB or VG001 virus (MOI = 1) for 24 hours. Cell lysates were prepared, ran on 12% SDS-PAGE gel, and transferred to PVDF membrane. The membrane was blotted with anti-human IL-12 antibody followed by HRP-conjugated anti-mouse IgG secondary antibody and the mage was detected and analyzed using Bio-Rad ImageLab system.

Fig. 11B-C shows that production of human IL-12 is upregulated after VG161-1215PLBh virus infection. LS174T or H460 tumour cells were infected with VG161-1215PLB or VG001 virus (MOI = 1) for 48 hours. Infected cell supernatants were harvested and bound to anti-human IL-12 capture antibody coated 96-well Immuno Maxisorp flat bottom plate. Binding was detected via a biotinylated anti-human IL-12 antibody, avidin-horseradish peroxidase (HRP), and 3,3',5,5'-Tetramethylbenzidine (TMB) substrate. Absorbance measurements were collected at 450 nm via a plate reader. The concentration of human IL-12 in cultured supernatants was calculated based on human IL-12 standard curve.

### EXAMPLE 6

### EXPRESSION OF IL-15 FOLLOWING INFECTION OF CELLS BY HVG161

In this Example, Western blot and ELISA data of IL-15 expression is shown.

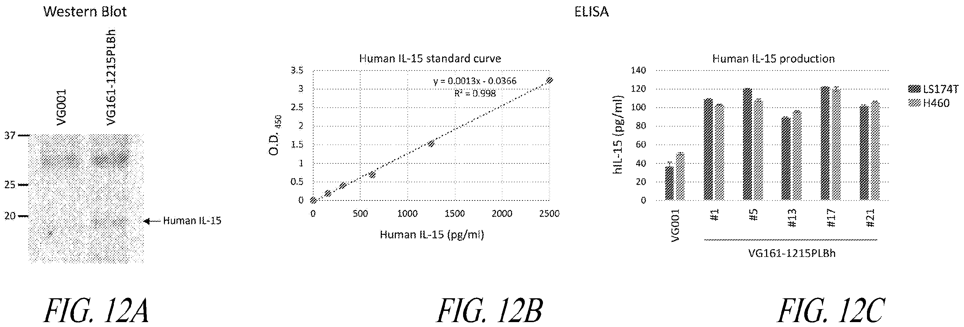

Fig. 12A shows Western Blot results after VG161-1215PLBh virus infection. H460 tumour cells were infected with VG161-1215PLB or VG001 virus (MOI = 1) for 24 hours. Cell lysates were prepared, ran on 12% SDS-PAGE gel, and transferred to PVDF membrane. The membrane was blotted with anti-human IL-15 antibody followed by HRP-conjugated anti-mouse IgG secondary antibody and the image was detected and analyzed using Bio-Rad ImageLab system.

Fig. 12B-C shows that production of human IL-15 is upregulated after VG161-1215PLBh virus infection. LS174T or H460 tumour cells were infected with VG161-1215PLB or VG001 virus (MOI = 1) for 48 hours. Infected cell supernatants were harvested and bound to anti-human IL-15 capture antibody coated 96-well Immuno Maxisorp flat bottom plate. Binding was detected via a biotinylated anti-human IL-15 antibody, avidin-horseradish peroxidase (HRP), and 3,3',5,5'-Tetramethylbenzidine (TMB) substrate. Absorbance measurements were collected at 450 nm via a plate reader. The concentration of human IL-15 in cultured supernatants was calculated based on human IL-15 standard curve.

### EXAMPLE 7

### EXPRESSION OF IGG4 FOLLOWING INFECTION OF CELLS BY HVG161

In this Example, Western blot and ELISA data of IgG4 expression is shown.

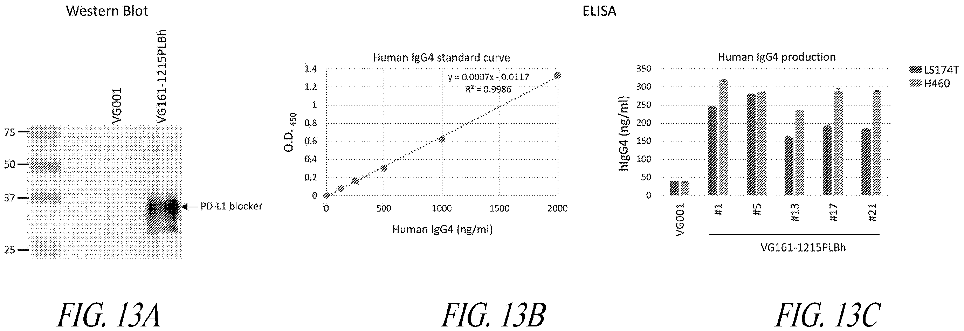

Fig. 13A shows Western Blot results after VG161-1215PLBh virus infection. H460 tumour cells were infected with VG161-1215PLB or VG001 virus (MOI = 1) for 24 hours. Cell lysates were prepared, ran on 12% SDS-PAGE gel, and transferred to PVDF membrane. The membrane was blotted with HRP-conjugated anti-human IgG antibody and the image was detected and analyzed using Bio-Rad ImageLab system.

Fig. 13B-C shows that production of human PD-L1 blocker (fused to human Fc domain) is upregulated after VG161-1215PLBh virus infection. LS174T or H460 tumour cells were infected with VG161-1215PLB or VG001 virus (MOI = 1) for 48 hours. Infected cell supernatants were harvested and bound to anti-human IgG4 capture antibody coated 96-well Immuno Maxisorp flat bottom plate. Binding was detected via a biotinylated anti-human IgG4 antibody, avidin-horseradish peroxidase (HRP), and 3,3',5,5'-Tetramethylbenzidine (TMB) substrate. Absorbance measurements were collected at 450 nm via a plate reader. The concentration of human IgG4 in cultured supernatants was calculated based on human IgG4 standard curve.

### EXAMPLE 8

### IN VITRO EFFICACY OF VIRUSES VG161 -PLBH AND VG161-15H

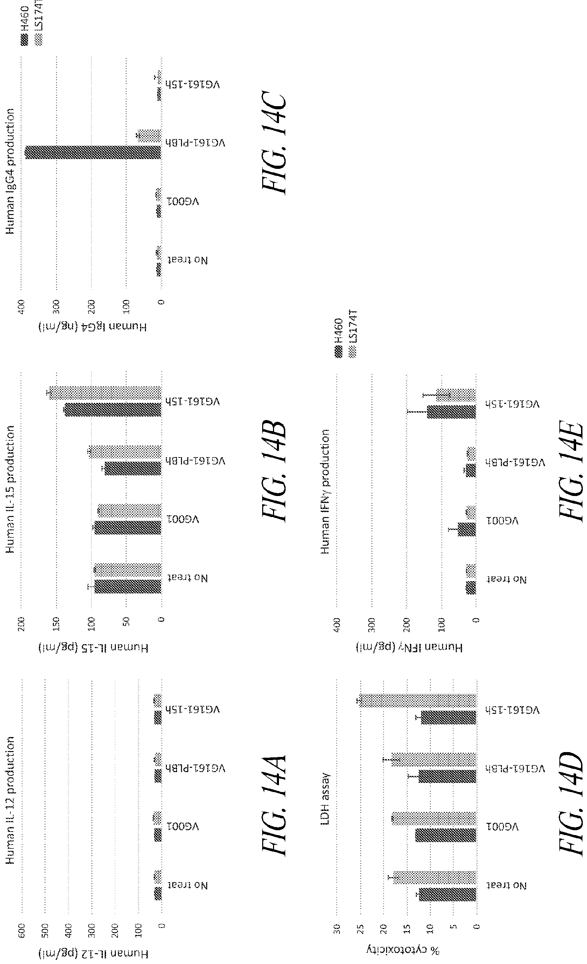

In this example, 3 x 10⁴ H460 or LS174T tumour cells were seeded into each well of 96-well plate and cultured at 37°C for overnight. Next day, seeded cells were infected with VG001 backbone, VG161-PLBh, or VG161-15h virus (MOI = 1) for 24 hours and the productions of human IL-12, human IL-15, and human IgG4 were assessed (Fig. 14A-C). 3 x 10⁵ human PBMCs were subsequently added into the culture and co-incubated for 24 hour to assess cytotoxicity by LDH assay (Fig. 14D) or 48 hours for human IFNg production by ELISA (Fig. 14E). For the cytotoxicity assay, percentage of cytotoxicity was calculated based on the following formula: [(actual reading - minimum release) / (Maximum release - minimum release)] x 100%. Supernatant harvested from tumour cells incubated with medium only was used as minimum release, and supernatant harvested from tumour cells incubated with lysis buffer was used as maximum release.

### EXAMPLE 9

### IN VITRO EFFICACY OF VARIOUS CONSTRUCTS

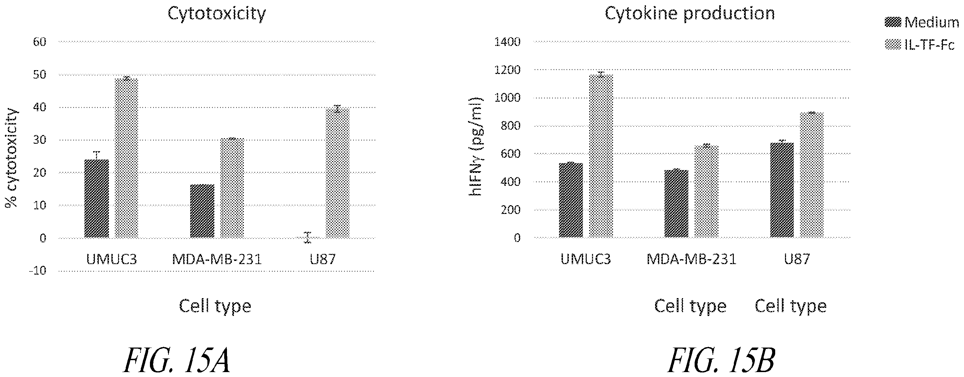

Figures 15A-15D show results of in vitro assays for various constructs.

Fig. 15A-15B show results of cell transfection with IL-TF-Fc plasmid carrying IL-12, IL-15, and PD-L1 blocker. In Figs. 15A-15B, different tumour cell lines were transfected with IL-TF-Fc plasmid DNA for 24 hours, and human PBMCs were subsequently added into the culture. Cell supernatants were harvested after 24 hours for quantification of cytotoxicity by LDH assay (Fig. 15A), and after 48 hours for detection of human IFNg production by ELISA assay (Fig. 15B).

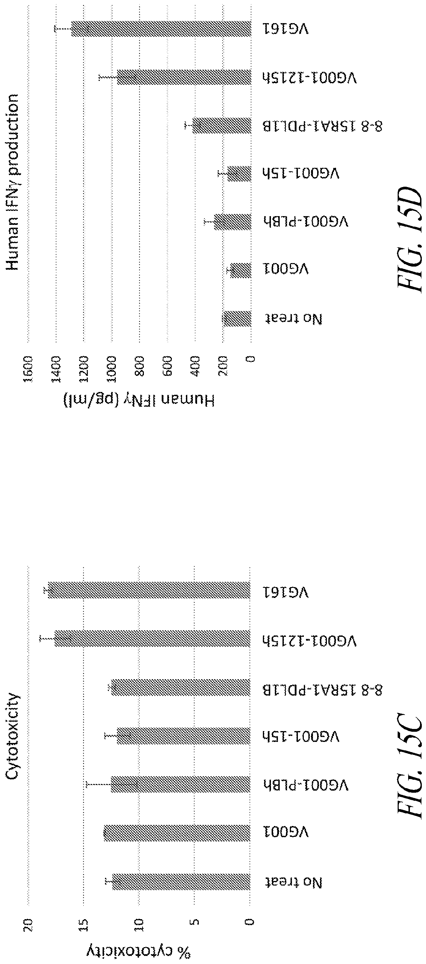

Figures 15C-15D show results of cell infection with a variety of mutant viruses including hVG161. Virally encoded IL12, IL15, and PD-L1 blocker synergistically enhance IFNg production and cytotoxicity. H460 tumour cells were seeded into each well of a 96-well plate and cultured at 37°C for overnight. Next day, seeded cells were infected with the indicated viruses at MOI = 1 for 24 hours. Human PBMCs were subsequently added into the culture and co-incubated for 24 hour to assess cytotoxicity by LDH assay (Figure 15C) or 48 hours for human IFNg production by ELISA (Figure 15D). For cytotoxicity assay, the percentage of cytotoxicity was calculated based on the following formula: [(actual reading - minimum release) / (Maximum release - minimum release)] x 100%. Supernatant harvested from tumour cells incubated with medium only was used as minimum release, and supernatant harvested from tumour cells incubated with lysis buffer was used as Maximum release.

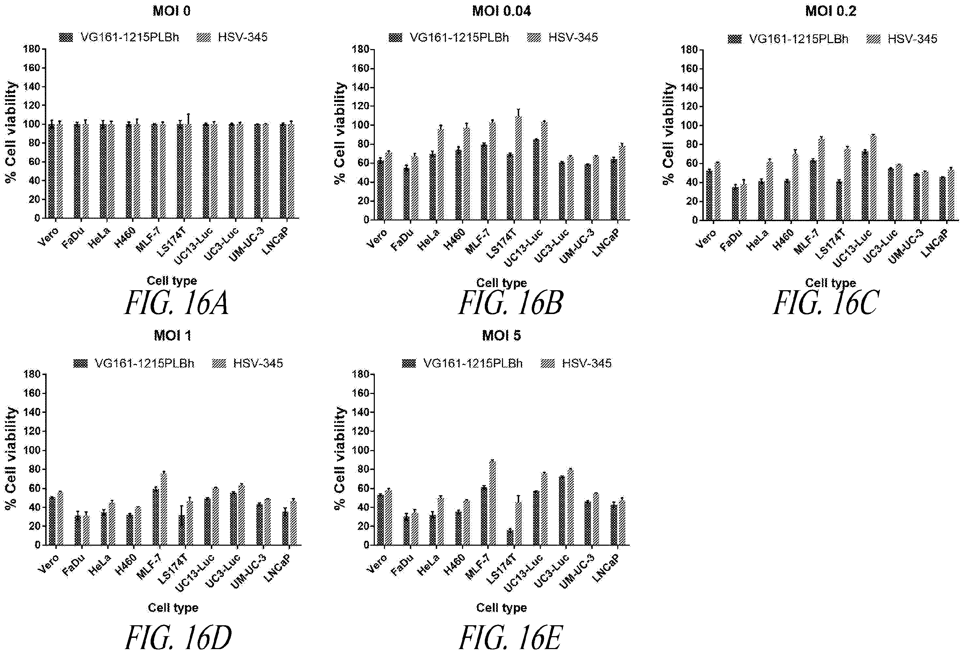

In Figs. 16A-16E, a panel of 9 different human tumor cell lines (plus Vero cells) was infected with VG161-1212PLBh (VG161h) and HSV-345 viruses at MOI 0, 0.04, 0.2, 1, and 5. Cell viability was quantified using MTT assay at 48 hours post infection.

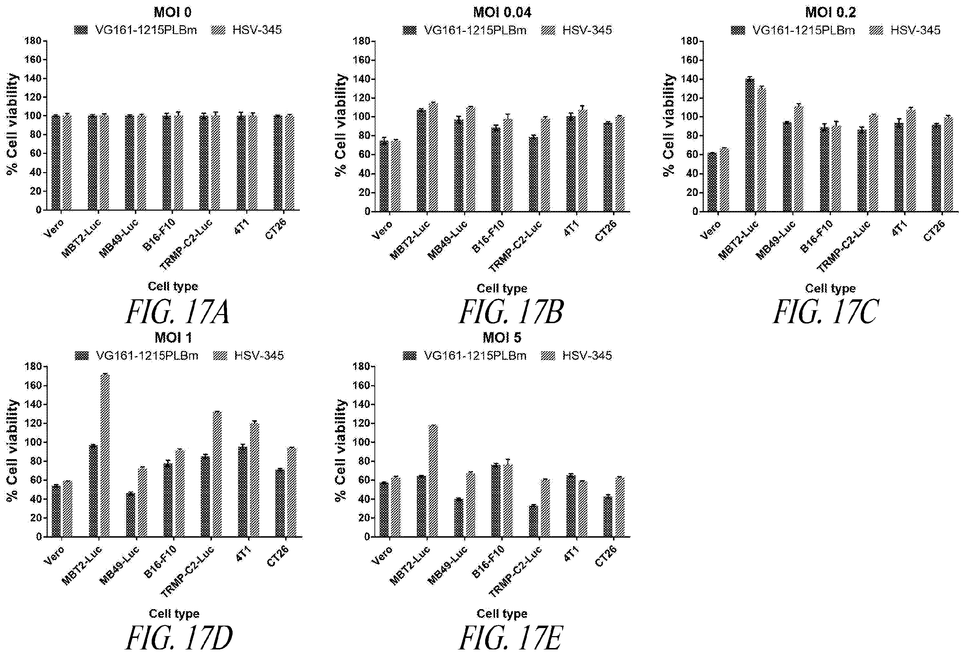

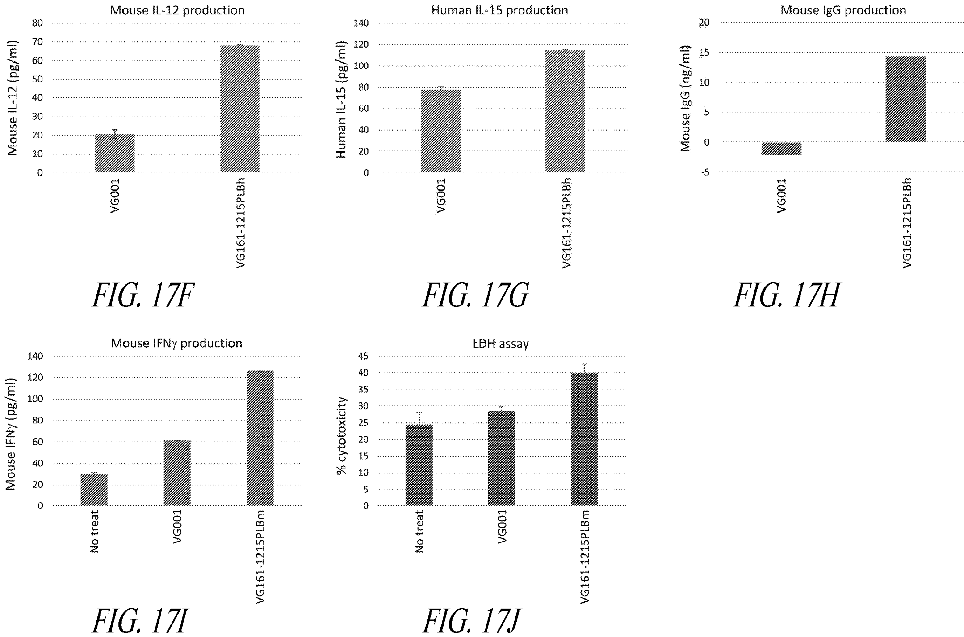

Figures 17A-17J show results of in vitro assays for various constructs. Figures 17A-17E show results of cell viability assays for mVG161 and HSV-345 on mouse tumor cell lines and Vero cell line; figures 17F-17J show the characterization of transgene expression following mVG161 or VG001 infection of CT26 mouse tumor cells.

In Figs. 17A-17E, a panel of 6 different mouse tumor cell lines (plus Vero cells) was infected with VG161m and HSV-345 viruses at MOI 0, 0.04, 0.2, 1, and 5. Cell viability was quantified using MTT assay at 48 hours post infection.

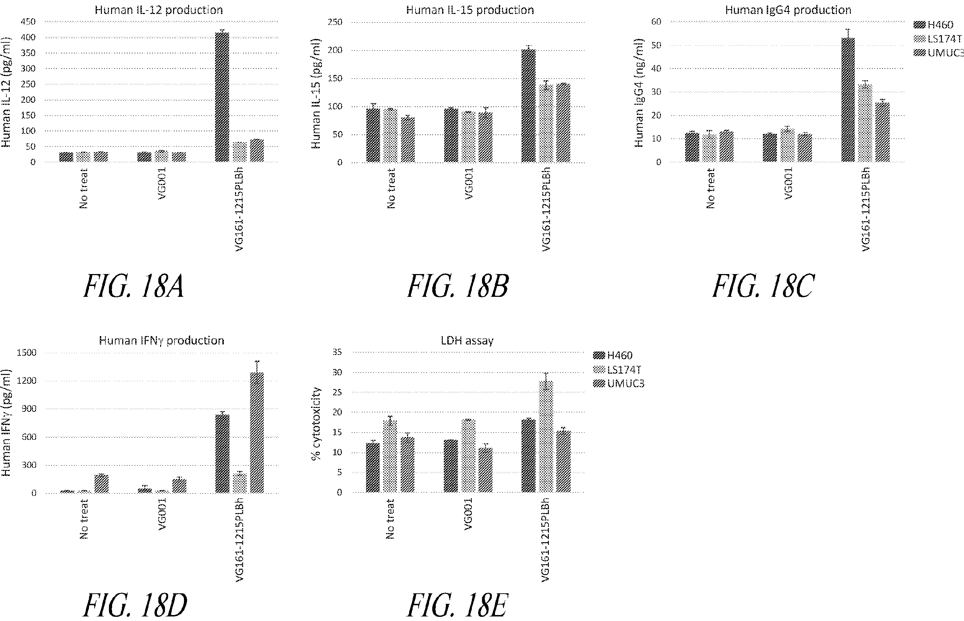

In Figs. 17F-17J, 3 x 10⁴ CT26 tumour cells were seeded into each well of 96-well plate and cultured at 37°C for overnight. Next day, seeded cells were infected with VG001 backbone or VG161-1215PLBm virus (MOI = 1) for 24 hours and the production of mouse IL-12, human IL-15, and mouse IgG was assessed (Fig. 18O). 3 x 10⁵ splenocytes from Balb/c mouse were subsequently added into the culture and co-incubated for 24 hour to assess cytotoxicity by LDH assay (Fig. 18P) or 48 hours for mouse IFNg production by ELISA (Fig. 18Q). For the cytotoxicity assay, percentage of cytotoxicity was calculated based on the following formula: [(actual reading - minimum release) / (Maximum release - minimum release)] x 100%. Supernatant harvested from tumour cells incubated with medium only was used as minimum release, and supernatant harvested from tumour cells incubated with lysis buffer was used as maximum release.

In Figs. 18A-18E, 3 x 10⁴ H460, LS174T, or UMUC3 tumor cells were seeded into each well of 96-well plate and cultured at 37°C for overnight. Next day, seeded cells were infected with VG001 backbone and VG161-1215h virus (MOI = 1) for 24 hours and the productions of human IL-12, human IL-15, and human IgG4 were assessed (18R). 3 x 10⁵ human PBMCs were subsequently added into the culture and co-incubated for 24 hour to assess cytotoxicity by LDH assay (18S) or 48 hours for human IFNγ production by ELISA (18T). For the cytotoxicity assay, percentage of cytotoxicity was calculated based on the following formula: [(actual reading - minimum release) / (Maximum release - minimum release)] x 100%. Supernatant harvested from tumor cells incubated with medium only was used as minimum release, and supernatant harvested from tumor cells incubated with lysis buffer was used as maximum release.

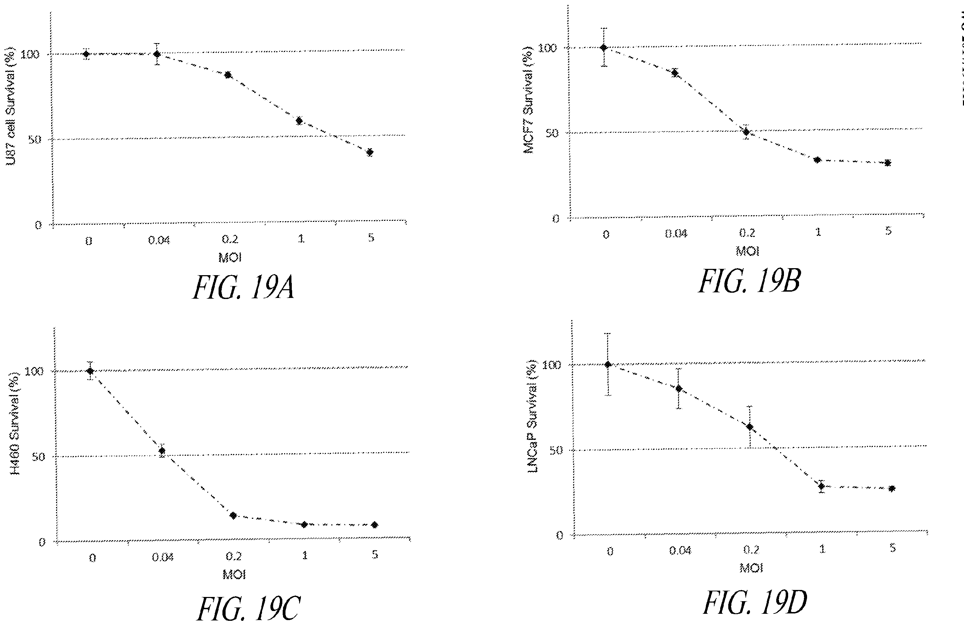

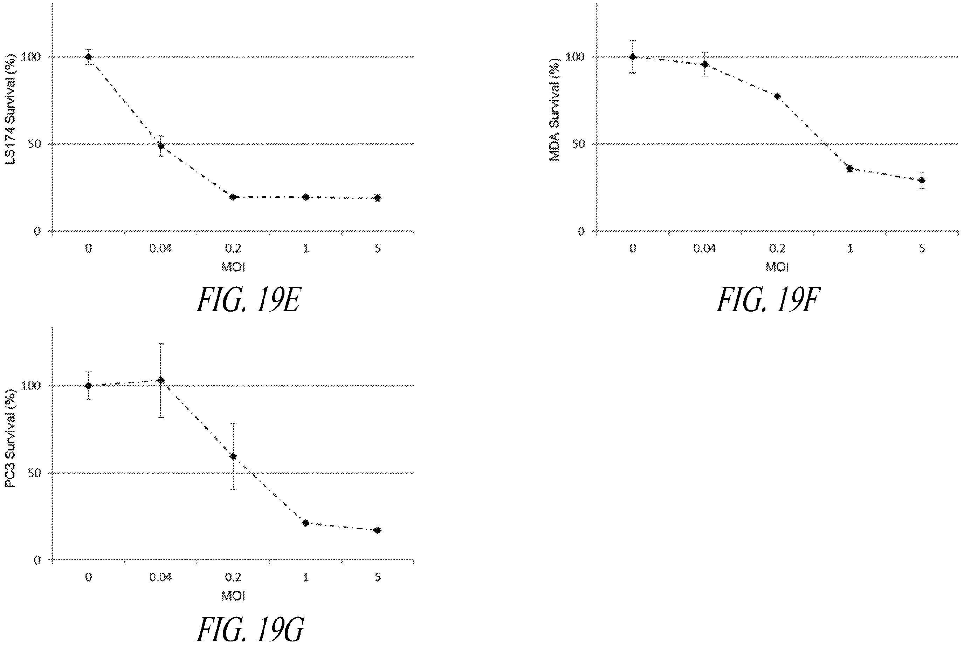

In Fig. 19A-19G, the antitumor effect of VG161-1215PLBh (hVG161) virus was evaluated in a variety human cancer cells including U87, MCF7, H460, LNCaP, LS174T, MDA, and PC3 at 72h post infection and MOIs ranging from 0 to 5. Cell survival percentage was quantified by MTT assay. The VG161-1215PLBh virus exhibits robust cell killing ability in all of the tested human tumor cell lines.

### EXAMPLE 11

### IN VIVO EFFICACY OF VG161 VIRAL CONSTRUCTS

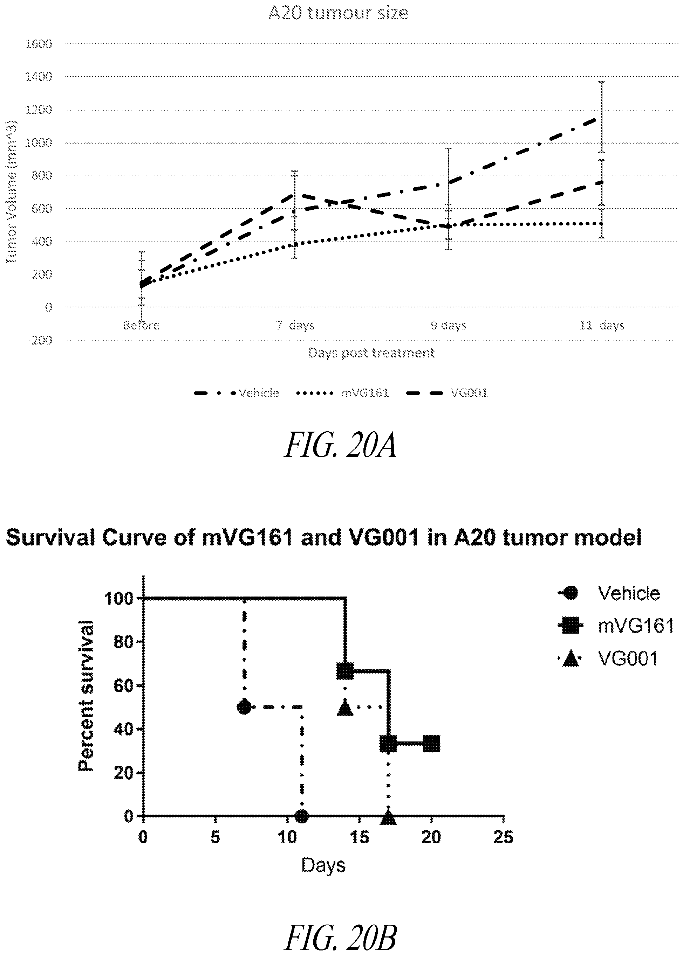

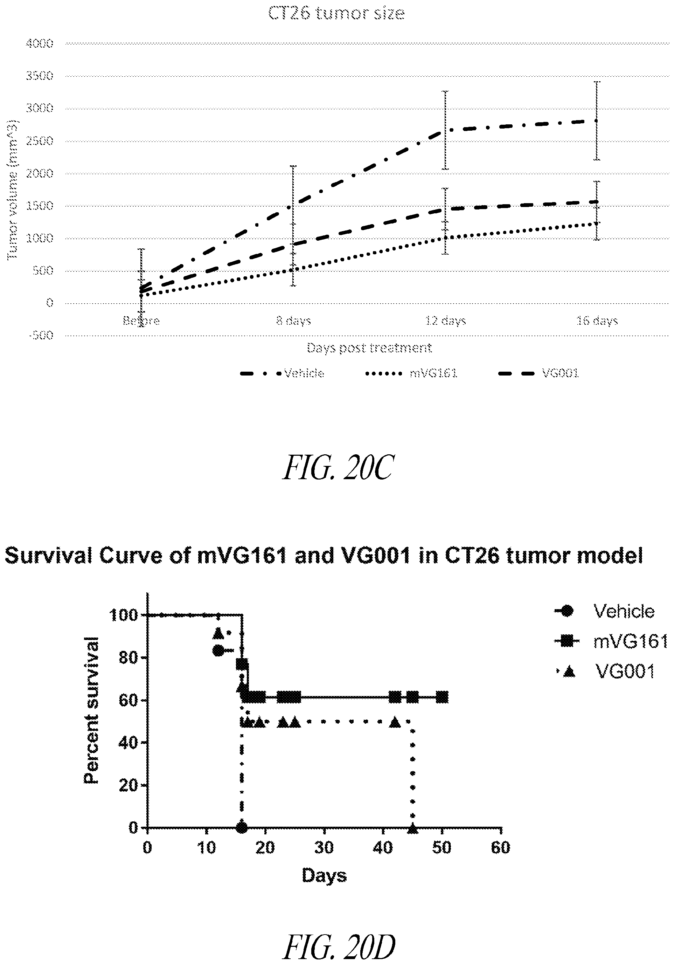

In Fig. 20A-B, BALB/c mice bearing A20 murine B-cell lymphoma tumors were injected 5 times intratumorally with a total of 1x10^7 PFU/mouse of either VG161-1215PLBm (mVG161) virus or VG001 backbone virus or with PBS (vehicle control). Tumor size measurements were performed at the indicated times post injection. Mice treated with VG161-1215PLBm exhibited a significant (P < 0.05) reduction in tumor volume compared to mice treated with PBS.

In Figs. 20C-D, BALB/c mice bearing CT26 murine colon carcinoma tumors were injected 5 times intratumorally with a total of 5×10^6 PFU/mouse of either VG161-1215PLBm (mVG161) virus or VG001 backbone virus or with PBS (vehicle control). Tumor size measurements were performed at the indicated times post injection. Mice treated with VG161-1215PLBm exhibited a significant (P < 0.05) reduction in tumor volume compared to mice treated with PBS.

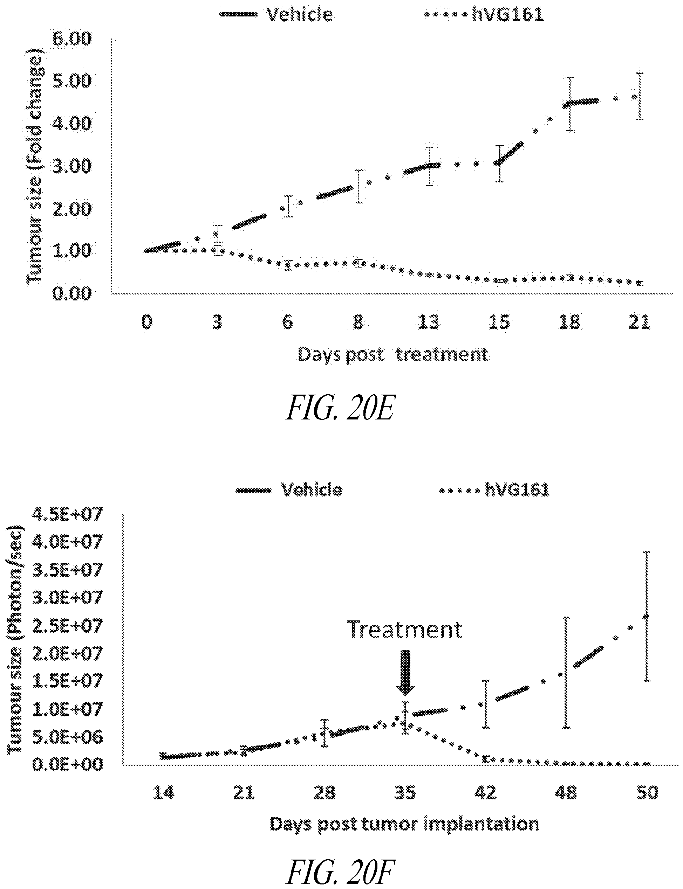

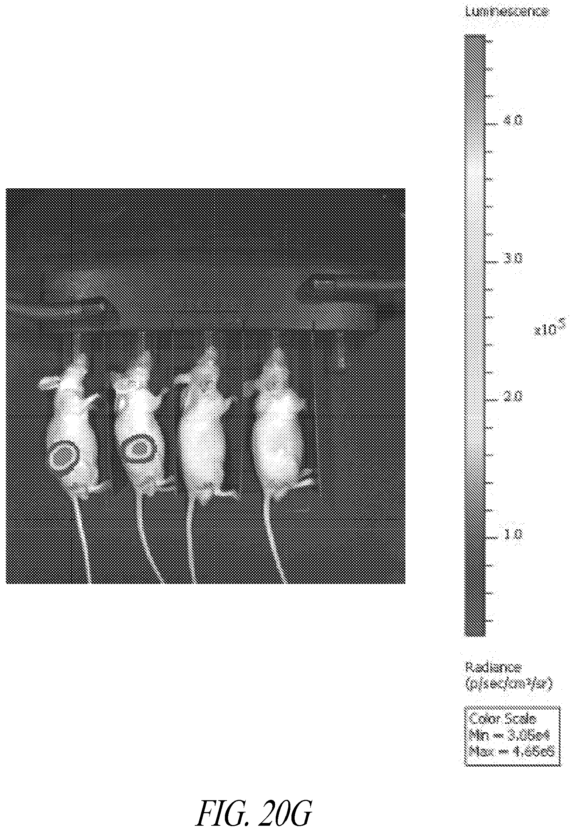

In Figs. 20E-G, oHSV treatment of xenograft human prostate tumors in mice was assessed. Twelve mice were implanted with LNCaP human prostate tumor cells in the right lower flank. At 35 days post implantation, a randomly selected group of 6 animals was injected twice intratumorally with a total of 5x10^7 PFU/mouse of VG161-1215PLBh (hVG161) virus, while the remaining 6 animals served as a vehicle control and were injected twice with an equivalent volume of PBS. Tumor size measurements were performed using two different methods. Caliper measurements are expressed as fold change in tumor volume at a given time point compared to the tumor volume at the time of virus or PBS injection (Figure 20E). Tumor-bearing mice treated with VG161-1215PLBh virus exhibited robust tumor shrinkage during the course of the study with over 50% reduction in tumor size at the end of 15 days, while vehicle-treated mice showed approximately 3-fold increases in tumor volume during the same time span. Tumor growth was also monitored using a whole animal bioluminescent imaging system (IVIS Imaging System; Xenogen, Mountain View, CA). Signal intensities were quantified as the sum of all detected photons per second (Figure 20F). Quantitative imaging of tumor growth using the IVIS system shows an even more dramatic reduction in tumor size in oHSV-treated animals compared to PBS-treated controls, with fluorescence dropping to undetectable levels by 50 days post tumor implantation (Figure 20G; two vehicle controls on left and two oHSV-treated mice on right).

### EXAMPLE 12

### REPLICATION OF HVG161 IN CELL LINES

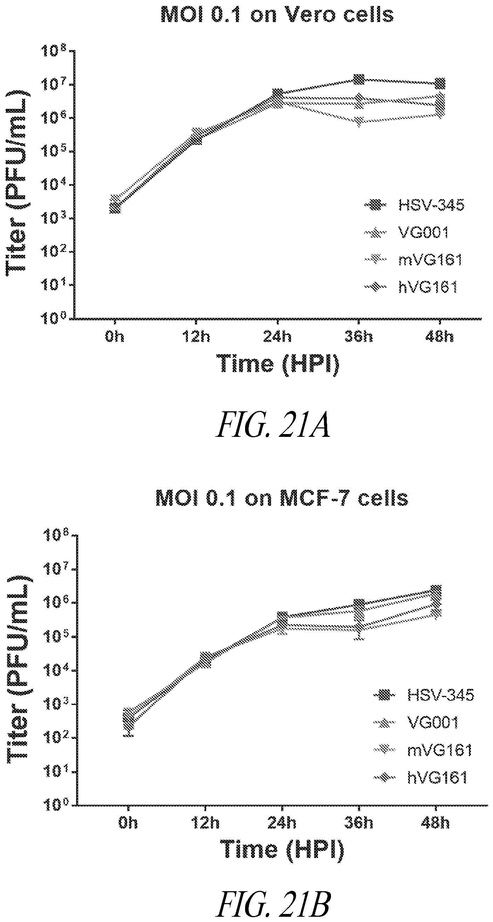

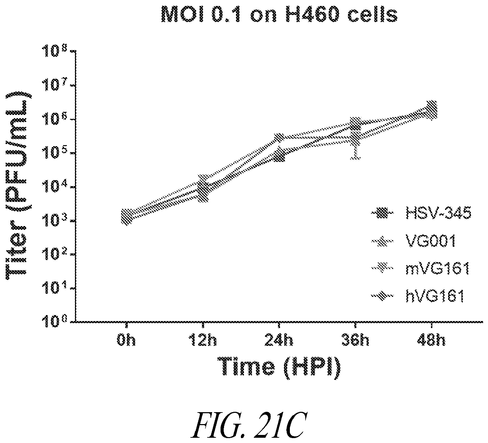

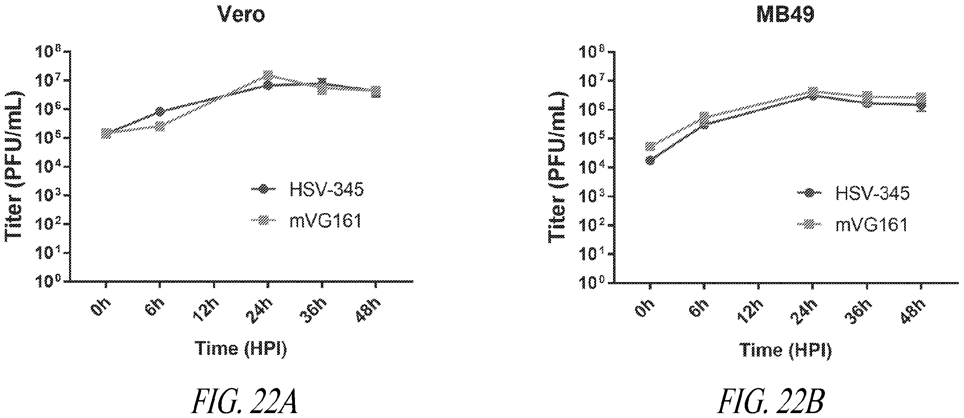

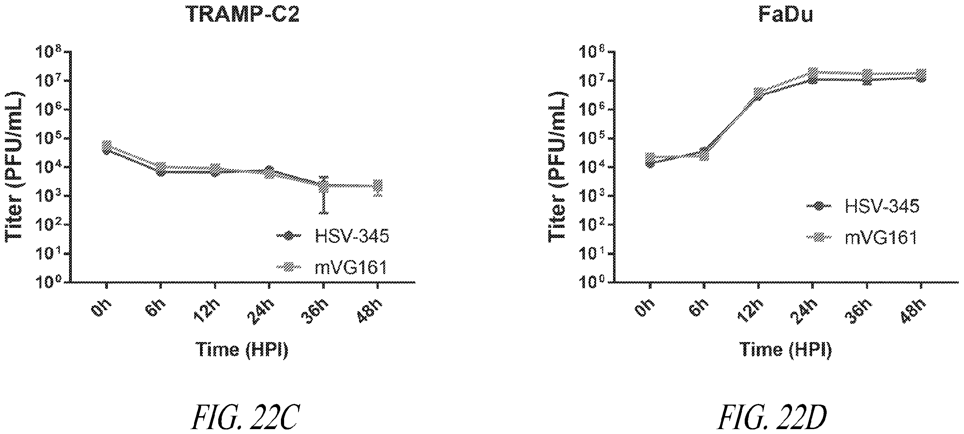

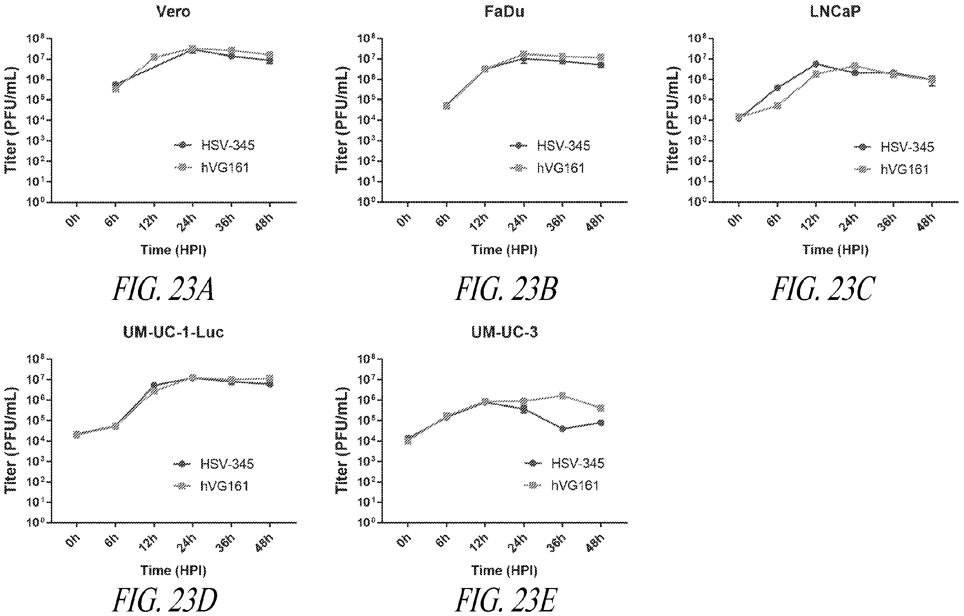

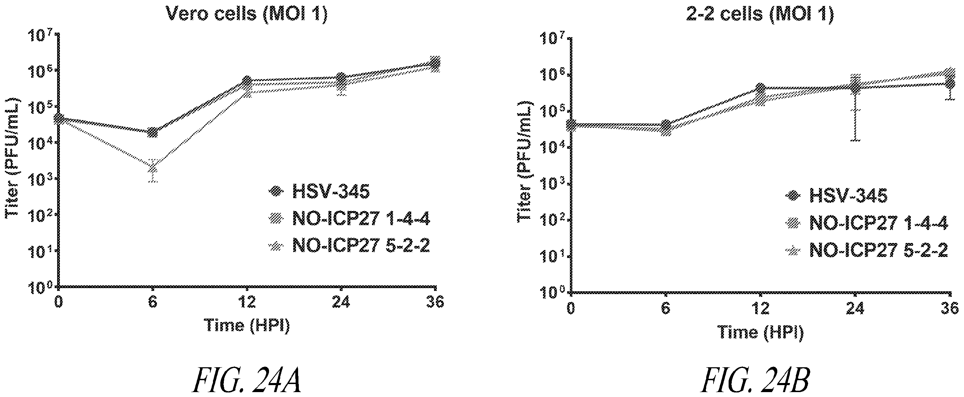

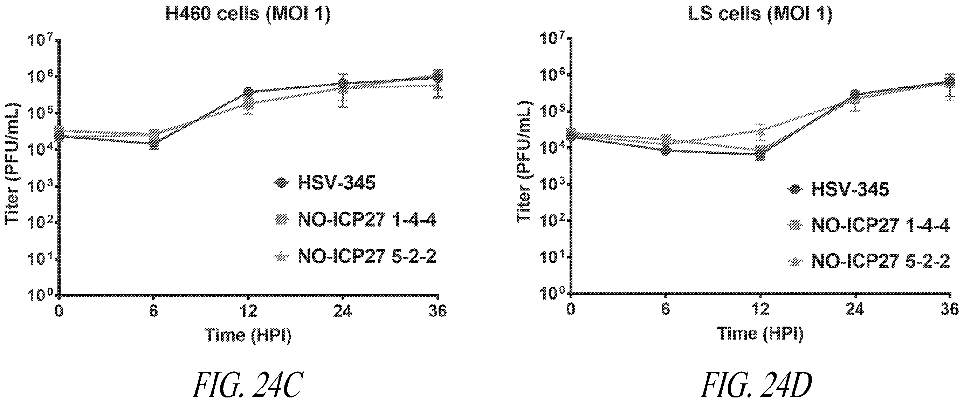

The growth curve and cytotoxicity data in Figures 21-24 show that hVG161 viruses replicate as well as the parental HSV-345 virus. These data also show that the viruses do not grow as well in mouse tumor cell lines compared to human cell lines, but HSV-1 is known to grow poorly in mouse cells.

### REFERENCES

1. Goldsten DJ and Weller SK, Herpes simplex virus type 1-induced ribonucleotide reductase activity is dispensable for virus growth and DNA synthesis: isolation and characterization of an ICP6 lacZ insertion mutant.J. Virol. 62, 196-205, 1988
2. Mineta T, Rabkin SD, Yazaki T, Hunter WD and Martuza RL. Attenuated multi-mutated herpes simplex virus-1 for the treatment of malignant gliomas. Nature medicine. 1995; 1(9):938-943.
3. Kooby DA, Carew JF, Halterman MW, Mack JE, Bertino JR, Blumgart LH, Federoff HJ and Fong Y. Oncolytic viral therapy for human colorectal cancer and liver metastases using a multi-mutated herpes simplex virus type-1 (G207). The FASEB journal. 1999; 13(11): 1325-1334.
4. Todo T, Martuza RL, Rabkin SD and Johnson PA. Oncolytic herpes simplex virus vector with enhanced MHC class I presentation and tumor cell killing. Proceedings of the National Academy of Sciences. 2001; 98(11):6396-6401.
5. Mace A, Ganly I, Soutar DS and Brown SM. Potential for efficacy of the oncolytic Herpes simplex virus 1716 in patients with oral squamous cell carcinoma. Head & neck. 2008; 30(8): 1045-1051.
6. Harrow S, Papanastassiou V, Harland J, Mabbs R, Petty R, Fraser M, Hadley D, Patterson J, Brown S and Rampling R. HSV1716 injection into the brain adjacent to tumour following surgical resection of high-grade glioma: safety data and long-term survival. Gene therapy. 2004; 11(22): 1648-1658.
7. Nakao A, Kasuya H, Sahin T, Nomura N, Kanzaki A, Misawa M, Shirota T, Yamada S, Fujii T and Sugimoto H. A phase I dose-escalation clinical trial of intraoperative direct intratumoral injection of HF10 oncolytic virus in non-resectable patients with advanced pancreatic cancer. Cancer gene therapy. 2011; 18(3): 167-175.
8. Fong Y, Kim T, Bhargava A, Schwartz L, Brown K, Brody L, Covey A, Karrasch M, Getrajdman G and Mescheder A. A herpes oncolytic virus can be delivered via the vasculature to produce biologic changes in human colorectal cancer. Molecular Therapy. 2009; 17(2):389-394.
9. Andtbacka RH, Kaufman HL, Collichio F, Amatruda T, Senzer N, Chesney J, Delman KA, Spitler LE, Puzanov I and Agarwala SS. Talimogene laherparepvec improves durable response rate in patients with advanced melanoma. Journal of Clinical Oncology. 2015:JCO. 2014.2058. 3377.
10. Gaston DC, Odom CI, Li L, Markert JM, Roth JC, Cassady KA, Whitley RJ and Parker JN. Production of bioactive soluble interleukin-15 in complex with interleukin-15 receptor alpha from a conditionally-replicating oncolytic HSV-1. PloS one. 2013; 8(11):e81768.
11. Parker JN, Gillespie GY, Love CE, Randall S, Whitley RJ and Markert JM. Engineered herpes simplex virus expressing IL-12 in the treatment of experimental murine brain tumors. Proceedings of the National Academy of Sciences. 2000; 97(5):2208-2213.
12. Passer BJ, Cheema T, Wu S, Wu C, Rabkin SD and Martuza RL. Combination of vinblastine and oncolytic herpes simplex virus vector expressing IL-12 therapy increases antitumor and antiangiogenic effects in prostate cancer models. Cancer gene therapy. 2013; 20(1): 17-24.
13. Carew JF, Kooby DA, Halterman MW, Kim S-H, Federoff HJ and Fong Y. A novel approach to cancer therapy using an oncolytic herpes virus to package amplicons containing cytokine genes. Molecular Therapy. 2001; 4(3):250-256.
14. Kambara H, Okano H, Chiocca EA and Saeki Y. An oncolytic HSV-1 mutant expressing ICP34. 5 under control of a nestin promoter increases survival of animals even when symptomatic from a brain tumor. Cancer Research. 2005; 65(7):2832-2839.
15. Fukuhara H, Ino Y, Kuroda T, Martuza RL and Todo T. Triple Gene-Deleted Oncolytic Herpes Simplex Virus Vector Double-Armed with Interleukin 18 and Soluble B7-1 Constructed by Bacterial Artificial Chromosome-Mediated System. Cancer research. 2005; 65(23): 10663-10668.

## Claims

1. An HSV vector comprising an NF-κB response element in a regulatory region of a viral gene that affects viral replication efficiency.

2. A HSV vector comprising an Oct-3/4-SOX2 response element in a regulatory region of viral gene that affects viral replication efficiency.

3. The vector of claims 1 or 2, wherein the viral gene encodes ICP4, ICP27, US11 or ICP8.

4. The vector of claim 1, wherein the NF-κB response element comprises from 1-15 tandem sequences of GGGAATTTCC or SEQ ID NO.s 10-34.

5. The vector of claim 4, wherein the tandem sequences are identical or a mix of identical and different sequences or all different sequences.

6. The vector of claim 4, wherein the NF-κB response element has the sequence GGGAATTTCCGGGGACTTTCCGGGAATTTCCGGGGACT TTCCGGGAATTTCC.

7. The vector of claim 2, where the OCT-3/4-SOX2 complex response element comprises SEQ ID NO: 2 or a variant thereof, wherein the variant has at least 90% identical nucleotides.

8. The HSV vector of claims 1-7, further comprising a sequence encoding IL12, IL15, OX40L, PDL-1 blocker or a PD-1 blocker.

9. An HSV vector according to claims 1-8 for use in a method of treating cancer, said method comprising administering the HSV vector to a patient in need.

10. An HSV vector according to claims 1-8 for use in a method of treating cancer stem cells and treatment-resistant cancer, said method comprising administering the HSV vector to a patient with a treatment resistant cancer, a cancer having cancer stem cells.

11. The HSV vector for use of claims 9-10, wherein the cancers are melanoma, colon cancer, liver cancer, lung cancer, breast cancer, pancreatic cancer, prostate cancer, brain cancer, or bladder cancer.

12. The HSV vector for use of claims 9-10, wherein the cancer comprises solid tumors.

## Patentansprüche

1. HSV-Vektor, der ein NF-κB-Reaktionselement in einer regulatorischen Region eines viralen Gens umfasst, das eine virale Replikationseffizienz beeinflusst.

2. HSV-Vektor, der ein Oct-3/4-SOX2-Reaktionselement in einer regulatorischen Region des viralen Gens umfasst, das eine virale Replikationseffizienz beeinflusst.

3. Vektor nach Anspruch 1 oder 2, wobei das virale Gen für ICP4, ICP27, US11 oder ICP8 codiert.

4. Vektor nach Anspruch 1, wobei das NF-κB-Reaktionselement von 1-15 Tandemsequenzen von GGGAATTTCC oder SEQ ID NO.s 10-34 umfasst.

5. Vektor nach Anspruch 4, wobei die Tandemsequenzen identisch sind oder eine Mischung aus identischen und unterschiedlichen Sequenzen oder alle unterschiedliche Sequenzen sind.

6. Vektor nach Anspruch 4, wobei das NF-κB-Reaktionselement die Sequenz GGGAATTTCCGGGGACTTTCCGGGAATTTCCGGGGACTTTCCGGGAATTTCC aufweist.

7. Vektor nach Anspruch 2, wobei das OCT-3/4-SOX2-Komplexreaktionselement SEQ ID NO: 2 oder eine Variante davon umfasst, wobei die Variante wenigstens 90 % identische Nucleotide aufweist.

8. HSV-Vektor nach den Ansprüchen 1-7, der ferner eine Sequenz umfasst, die für IL12, IL15, OX40L, PDL-1-Blocker oder einen PD-1-Blocker codiert.

9. HSV-Vektor nach den Ansprüchen 1-8 zur Verwendung in einem Verfahren zum Behandeln von Krebs, wobei das Verfahren ein Verabreichen des HSV-Vektors an einen bedürftigen Patienten umfasst.

10. HSV-Vektor nach den Ansprüchen 1-8 zur Verwendung in einem Verfahren zum Behandeln von Krebsstammzellen, wobei das Verfahren das Verabreichen des HSV-Vektors an einen Patienten mit einem behandlungsresistenten Krebs umfasst, wobei ein Krebs Krebsstammzellen aufweist.

11. HSV-Vektor zur Verwendung nach den Ansprüchen 9-10, wobei es sich bei den Krebsarten um Melanom, Dickdarmkrebs, Leberkrebs, Lungenkrebs, Brustkrebs, Bauchspeicheldrüsenkrebs, Prostatakrebs, Hirnkrebs oder Blasenkrebs handelt.

12. HSV-Vektor zur Verwendung nach den Ansprüchen 9-10, wobei der Krebs solide Tumore umfasst.

## Revendications

1. Vecteur HSV comprenant un élément de réponse NF-KB dans une région régulatrice d'un gène viral qui affecte l'efficacité de la réplication virale.

2. Vecteur HSV comprenant un élément de réponse Oct-3/4-SOX2 dans une région régulatrice du gène viral qui affecte l'efficacité de la réplication virale.

3. Vecteur selon les revendications 1 ou 2, dans lequel le gène viral code pour ICP4, ICP27, US11 ou ICP8.

4. Vecteur selon la revendication 1, dans lequel l'élément de réponse NF-KB comprend de 1 à 15 séquences en tandem de GGGAATTTCC ou SEQ ID NO. 10-34.

5. Vecteur selon la revendication 4, dans lequel les séquences en tandem sont identiques ou un mélange de séquences identiques et différentes ou de toutes les séquences différentes.

6. Vecteur selon la revendication 4, dans lequel l'élément de réponse NF-KB a la séquence GGGAATTTCCGGGGACTTTCCGGGAATTTCCGGGGACT TTCCGGGAATTTCC.

7. Vecteur selon la revendication 2, où l'élément de réponse complexe OCT-3/4-SOX2 comprend SEQ ID NO : 2 ou un variant de celui-ci, dans lequel le variant a au moins 90 % de nucléotides identiques.

8. Vecteur HSV selon les revendications 1 à 7, comprenant en outre une séquence codant pour IL12, IL15, OX40L, un bloqueur de PDL-1 ou un bloqueur de PD-1.

9. Vecteur HSV selon les revendications 1 à 8 destiné à être utilisé dans une méthode de traitement du cancer, ladite méthode comprenant l'administration du vecteur HSV à un patient dans le besoin.

10. Vecteur HSV selon les revendications 1 à 8 pour une utilisation dans une méthode de traitement des cellules souches cancéreuses et du cancer résistant au traitement, ladite méthode comprenant l'administration du vecteur HSV à un patient atteint d'un cancer résistant au traitement, un cancer ayant des cellules souches cancéreuses.

11. Vecteur HSV à utiliser selon les revendications 9 à 10, dans lequel les cancers sont le mélanome, le cancer du côlon, le cancer du foie, le cancer du poumon, le cancer du sein, le cancer du pancréas, le cancer de la prostate, le cancer du cerveau ou le cancer de la vessie.

12. Vecteur HSV à utiliser selon les revendications 9 à 10, dans lequel le cancer comprend des tumeurs solides.
